(19) Europäisches Patentamt
European Patent Office
Office européen des brevets

(11) EP 3 173 416 B1

(12) EUROPEAN PATENT SPECIFICATION

(45) Date of publication and mention of the grant of the patent:
**14.11.2018 Bulletin 2018/46**

(21) Application number: **15306858.0**

(22) Date of filing: **24.11.2015**

(51) Int Cl.:
*C07F 5/02* (2006.01)
*A61B 5/00* (2006.01)
*A61K 49/00* (2006.01)
*G01N 31/00* (2006.01)
*G01N 21/00* (2006.01)

(54) **BORONDIFLUORIDE COMPLEXES OF CURCUMINOID COMPOUNDS, METHOD OF PREPARATION AND USES THEREOF**

BORDIFLUORIDKOMPLEXE VON CURCUMINOIDVERBINDUNGEN, VERFAHREN ZUR HERSTELLUNG UND VERWENDUNGEN DAVON

COMPLEXES BORÉS DIFLUORIDES DE COMPOSÉS CURCUMINOÏDES, PROCÉDÉ DE PRÉPARATION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**31.05.2017 Bulletin 2017/22**

(73) Proprietors:
- **Université d'Aix Marseille**
  **13007 Marseille 7 (FR)**
- **Centre National de la Recherche Scientifique**
  **75016 Paris (FR)**

(72) Inventors:
- **ZABOROVA, Elena**
  **13008 MARSEILLE (FR)**
- **FAGES, Frédéric**
  **83110 SANARY SUR MER (FR)**
- **D'ALEO, Anthony**
  **13009 MARSEILLE (FR)**

(74) Representative: **Lavoix**
**2, place d'Estienne d'Orves**
**75441 Paris Cedex 09 (FR)**

(56) References cited:
- **KRAVTSOV, IVAN V. ET AL: "Boron-chelate assisted synthesis of di[pyrazol-3(5)-yl]methane and 1,1',2,2'-tetra[pyrazol-3(5)-yl]ethane", MENDELEEV COMMUNICATIONS, vol. 19, no. 1, 2009, pages 27-29, XP002757637, ISSN: 0959-9436, DOI: 10.1016/J.MENCOM.2009.01.011**
- **FU, HUALONG ET AL: "Highly Sensitive Near-Infrared Fluorophores for in Vivo Detection of Amyloid-.beta. Plaques in Alzheimer's Disease", JOURNAL OF MEDICINAL CHEMISTRY, vol. 58, no. 17, 11 August 2015 (2015-08-11), pages 6972-6983, XP002757638, ISSN: 0022-2623, DOI: 10.1021/ACS.JMEDCHEM.5B00861**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 173 416 B1

## Description

The present invention relates to new borondifluoride complexes of curcuminoid compounds with an enhanced fluorescence quantum yield and emission, and their uses as fluorophore in various fields such as bioimaging, therapeutics, theranostics, display and telecommunication technologies, photovoltaics. The preparation said compounds is also described.

Compounds displaying high molar absorption coefficients, large two-photon absorption cross sections, high luminescence quantum yields in solution and in the solid state, large Stokes shifts, high thermal and photochemical stability represent attractive candidates as fluorescent reporters. Especially, dyes that emit in the near infrared (NIR, wavelengths longer than 700 nm according to The International Commission on Illumination) using two-photon excitation in the NIR region are of great interest for use in cell imaging because both excitation and detection operate in the biological transparency window.

Among the many classes of organic dyes, boron complexes, such as borondipyrromethene (BODIPY) compounds, are particularly attractive. In addition of the interesting optical properties in solution, many borondifluoride complexes (other than BODIPY) have also been shown to yield rather efficient photoluminescent behavior in the solid state. In particular, compounds deriving from acetylacetonate ligand have shown interesting properties such as high two-photon absorption cross sections, mechano-fluorochromic behaviors and efficient NIR emissions that led to their use for cells imaging or as sensors of volatile acid/base, fluorescent reporters for amyloid, optical sensors for anaerobic environment and electron donors in solar cells.

Recently, curcuminoid structures containing the borondifluoride unit have been shown as efficient emitters that fulfill many of the previous requirements. However, this work also led to the conclusion that solid-state fluorescence of $BF_2$ complexes of curcuminoids arose from highly stacked chromophores. Such interactions could explain the emission occurring in the NIR but inherently induce efficient face-to-face quenching, which limit the fluorescence quantum yield ($\Phi_f$) to *ca.* 5 % (A. D'Aléo, D. Gachet, V. Heresanu, M. Giorgi and F. Fages, Chem. Eur. J., 2012, 18, 12764-12772; G. Bai, C. Yu, C. Cheng, E. Hao, Y. Wei, X. Mu and L. Jiao, Org. Biomol. Chem., 2014, 12, 1618-1626). Highly sensitive near-infrared fluorophores for in vivo detection of amyloid-β plaques in Alzheimer's disease are disclosed by Hualong Fu et al. in J. Med.Chem. 2015, 58, 6972-6983.

There exists a real need for new compounds with improved absorption ability and fluorescence quantum yield in solution and in the solid state.

It is an aim of the present invention to specifically meet these needs by providing a compound of general formula (I):

$$Q^2\text{-}Q^1\text{-}Q^3 \qquad (I)$$

wherein

- $Q^1$ represented by formula (II)

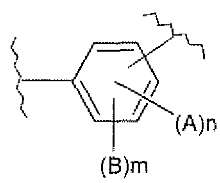

(II)

wherein

- $Q^1$ is substituted with $Q^2$ and $Q^3$,
- A is a $C_1$-$C_{12}$ alkoxy group,
- B is a difluoroboron beta-diketone of formula (III)

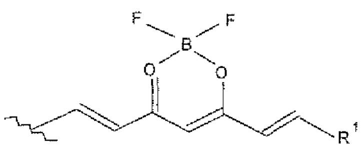

(III)

- n is 0, 1, 2, 3 or 4,

- m is 0 or 1; or

▪ $Q^1$ is represented by formula (IV)

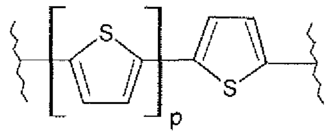

(IV)

wherein

- $Q^1$ is substituted with $Q^2$ and $Q^3$,
- p is 0, 1 or 2;

▪ $Q^2$ is a difluoroboron beta-diketone of formula (V)

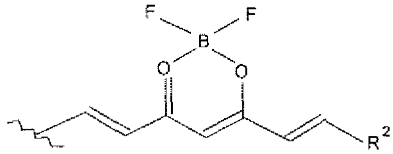

(V)

▪ $Q^3$ is a difluoroboron beta-diketone of formula (VI)

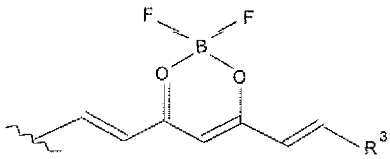

(VI)

▪ $R^1$, $R^2$ and $R^3$, are each independently chosen among:

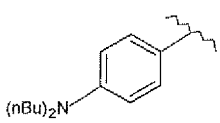 , 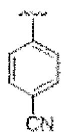 , 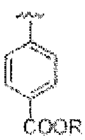 , 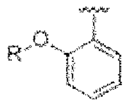 , 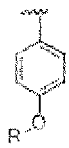 , 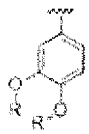 , 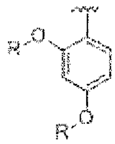 , 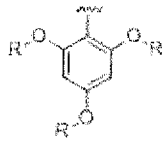 ,

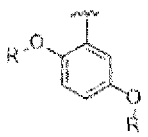 , 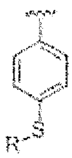 , 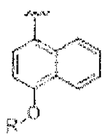 , 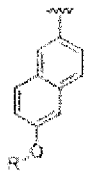 , 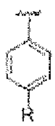 , 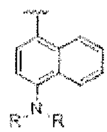 , 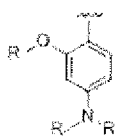 , 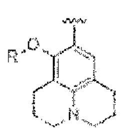 , 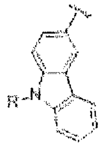 ,

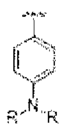 , 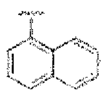 , 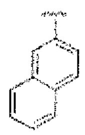 , 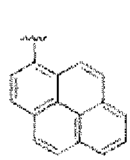 , 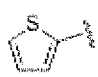 , 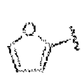 , 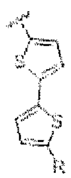 , 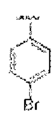 , 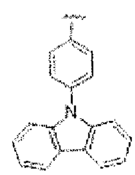 ,

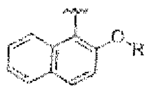, 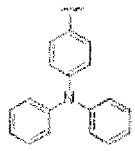

with R being each independently a hydrogen atom, a $C_1$-$C_{12}$ alkyl group.

Preferably, $R^1$, $R^2$ and $R^3$, are each independently chosen:

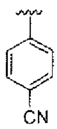 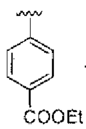 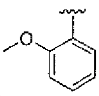 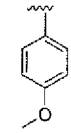 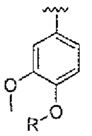 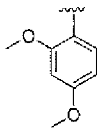 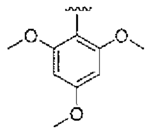 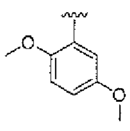 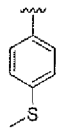

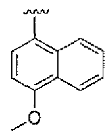 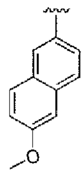 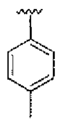 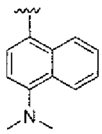 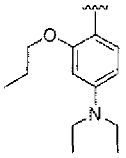 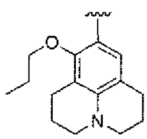 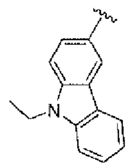 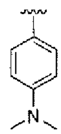

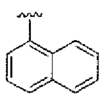 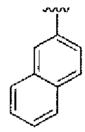 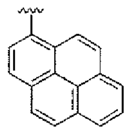 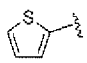 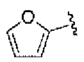 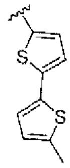 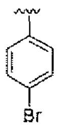 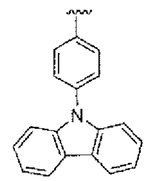

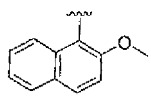 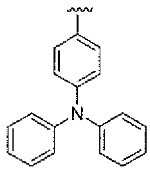 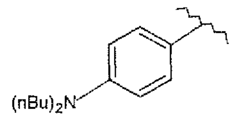

with R representing a methyl group.

The compounds of the invention exhibit enhanced optical properties in solution, such as high optical brightnesses obtained at one- and two-photon excitation. UV/visible absorption of solid-state particles comprising the compounds of the invention formed in water solution reveals that these compounds are strongly aggregated and fluorescence spectroscopy shows they are emissive in the NIR with enhanced fluorescence quantum yields in comparison with the compounds of prior art.

In the compounds of the invention, when $Q^1$ is represented by formula (II)

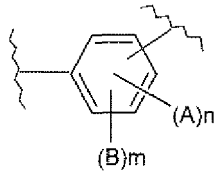

(II)

and n is 2, 3 or 4, the alkoxy groups A may be identical or different.

For the purposes of the present invention, the term "alkyl group" is understood to mean, an optionally substituted, saturated and linear, branched or cyclic carbon-comprising radical comprising from 1 to 12 carbon atoms, for example 1 to 8 carbon atoms. Mention may be made, as saturated and linear or branched alkyl, for example, of the methyl, ethyl,

propyl, butyl, pentyl, hexyl, octyl, nonyl, decyl, undecyl and dodecanyl radicals and their branched isomers. Mention may be made, as cyclic alkyl, of the cyclopropyl, cyclobutyl, cyclopentyl, cyclohexyl, bicyclo[2.1.1]hexyl and bicyclo[2.2.1]heptyl radicals. The alkyl group, within the meaning of the invention, can optionally be substituted by one or more hydroxyl groups, one or more alkoxy groups, one or more halogen atoms chosen from the fluorine, chlorine, bromine and iodine atoms, one or more nitro ($-NO_2$) groups, one or more nitrile (-CN) groups or one or more aryl groups, with the alkoxy and aryl groups as defined in the context of the present invention.

The term "aryl" denotes generally an aromatic cyclic substituent comprising from 6 to 20 carbon atoms, for example 6 to 10. In the context of the invention, the aryl group can be mono- or polycyclic. Mention may be made, by way of indication, of the phenyl, benzyl and naphthyl groups. The aryl group can optionally be substituted by one or more hydroxyl groups, one or more alkoxy groups, one or more halogen atoms chosen from the fluorine, chlorine, bromine and iodine atoms, one or more nitro ($-NO_2$) groups, one or more nitrile (-CN) groups or one or more alkyl groups, with the alkoxy and alkyl groups as defined in the context of the present invention.

The term "heteroaryl" denotes generally an aromatic mono- or polycyclic substituent comprising from 5 to 10 members, including at least 2 carbon atoms, and at least one heteroatom chosen from nitrogen, oxygen, boron, silicon, phosphorus or sulfur. The heteroaryl group can be mono- or polycyclic. Mention may be made, by way of indication, of the furyl, benzofuranyl, pyrrolyl, indolyl, isoindolyl, azaindolyl, thiophenyl, benzothiophenyl, pyridyl, quinolinyl, isoquinolyl, imidazolyl, benzimidazolyl, pyrazolyl, oxazolyl, isoxazolyl, benzoxazolyl, thiazolyl, benzothiazolyl, isothiazolyl, pyridazinyl, pyrimidinyl, pyrazinyl, triazinyl, cinnolinyl, phthalazinyl and quinazolinyl groups. The heteroaryl group can optionally be substituted by one or more hydroxyl groups, one or more alkoxy groups, one or more halogen atoms chosen from the fluorine, chlorine, bromine and iodine atoms, one or more nitro ($-NO_2$) groups, one or more nitrile (-CN) groups, one or more aryl groups or one or more alkyl groups, with the alkyl, alkoxy and aryl groups as defined in the context of the present invention.

The term "alkoxy group" means an alkyl group as defined above, bonded via an oxygen atom (-O-alkyl).

The term "halogen atom" is understood to mean an atom chosen from the fluorine, chlorine, bromine or iodine atoms.

In the compounds of formula (I), when $Q_1$ is

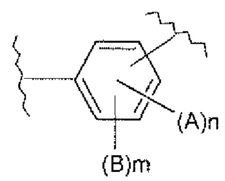

$Q_2$ and $Q_3$ may be in ortho, meta or para position.

According to a first embodiment of the invention, the compound of general formula (I) is a compound as defined above, wherein:

- $Q^1$ is represented by formula (IIa)

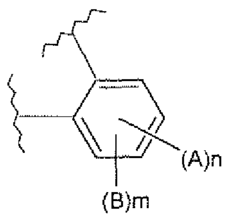

(IIa)

wherein

- $Q^1$ is substituted with $Q^2$ and $Q^3$ in ortho position,
- A is a $C_1$-$C_{12}$ alkoxy group,
- B is a difluoroboron beta-diketone of formula (III),

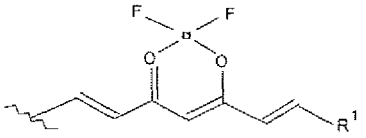 (III)

- n is 0, 1, 2, 3 or 4,
- m is 0 or 1; and

- $Q^2$ is a difluoroboron beta-diketone of formula (V)

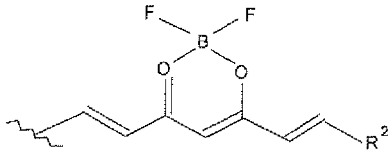 (V)

- $Q^3$ is a difluoroboron beta-diketone of formula (VI)

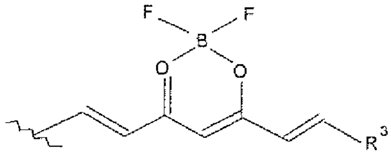 (VI)

- $R^1$, $R^2$ and $R^3$, are each independently chosen among:

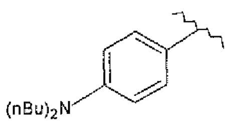, 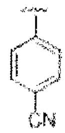, 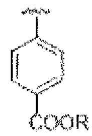, 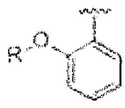, 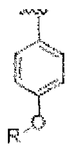, 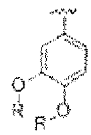, 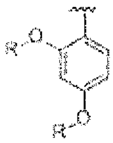, 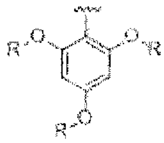,

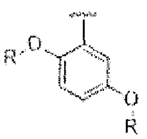, 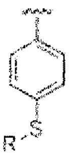, 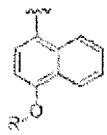, 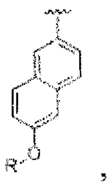, 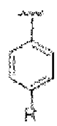, 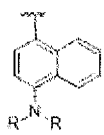, 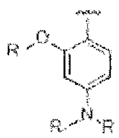, 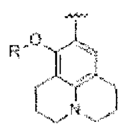, 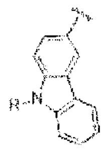,

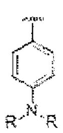, 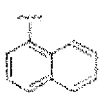, 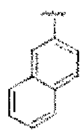, 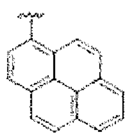, 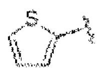, 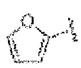, 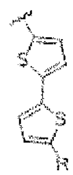, 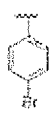, 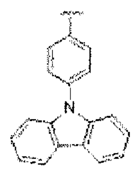,

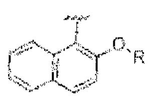, 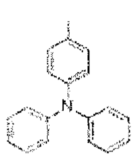

with R being each independently a hydrogen atom, a $C_1$-$C_{12}$ alkyl group.

According to a second embodiment of the invention, the compound of general formula (I) is a compound as defined above, wherein

- $Q^1$ is represented by formula (IIa)

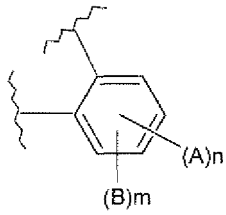

(IIa)

wherein

- $Q^1$ is substituted with $Q^2$ and $Q^3$ in ortho position,
- n=m=0; and

- $Q^2$ is a difluoroboron beta-diketone of formula (V)

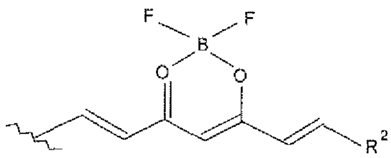

(V)

- $Q^3$ is a difluoroboron beta-diketone of formula (VI)

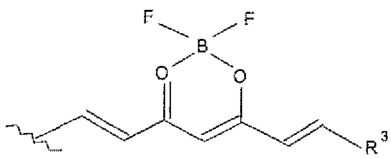

(VI)

with $R^2$ and $R^3$ being each independently chosen among:

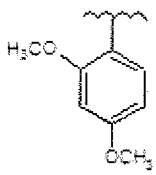
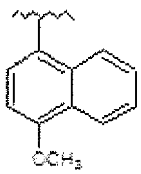
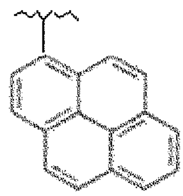
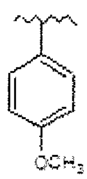

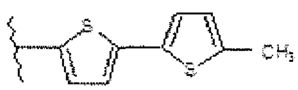
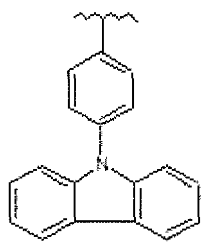
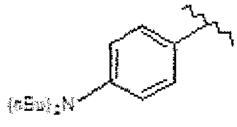

Examples of the first and second embodiments of the invention, are compounds 1 and 2:

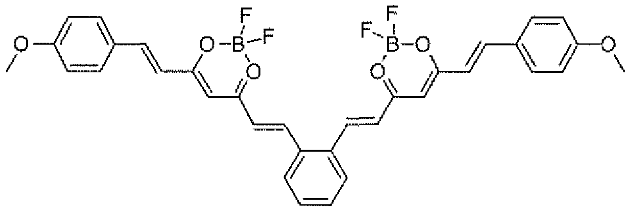

Compound 1

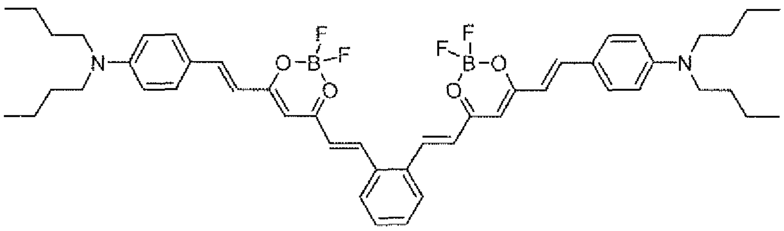

Compound 2

According to a third embodiment of the present invention, the compound of general formula (I) is a compound as defined above, wherein:

- $Q^1$ is represented by formula (IIb)

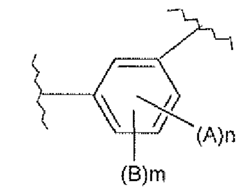

(IIb)

wherein

- $Q^1$ is substituted with $Q^2$ and $Q^3$ in meta position,
- A is a $C_1$-$C_{12}$ alkoxy group,
- B is a difluoroboron beta-diketone of formula (III)

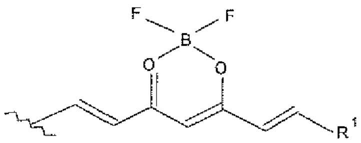

(III)

- n is 0, 1, 2, 3 or 4,
- m is 0 or 1; and

- $Q^2$ is a difluoroboron beta-diketone of formula (V)

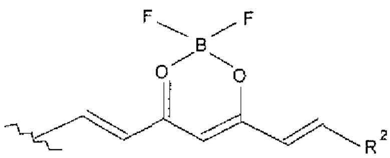

(V)

- $Q^3$ is a difluoroboron beta-diketone of formula (VI)

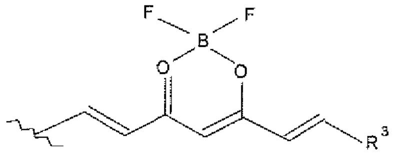

(VI)

- $R^1$, $R^2$ and $R^3$, are each independently chosen among:

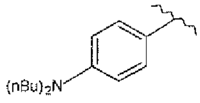, 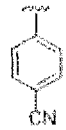, 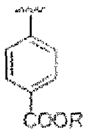, 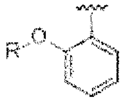, 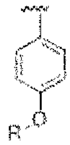, 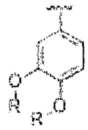, 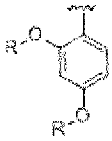, 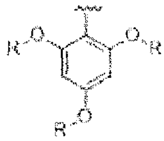,

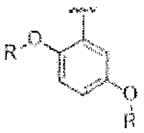, 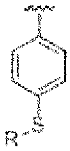, 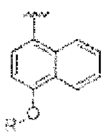, 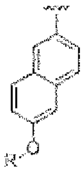, 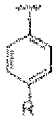, 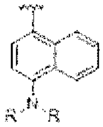, 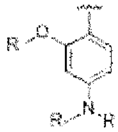, 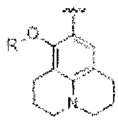, 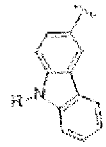,

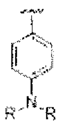, 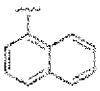, 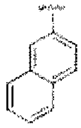, 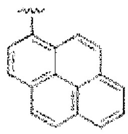, 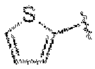, 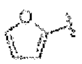, 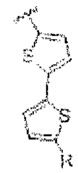, 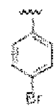, 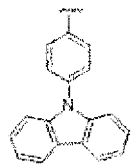,

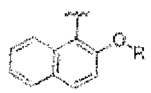, 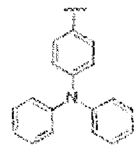

with R being each independently a hydrogen atom, a $C_1$-$C_{12}$ alkyl group.

According to a fourth embodiment of the present invention, the compound of general formula (I) is a compound as defined above, wherein:

- $Q^1$ is represented by formula (IIb)

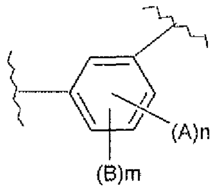

(IIb)

wherein

- $Q^1$ is substituted with $Q^2$ and $Q^3$ in meta position,
- A is a $C_1$-$C_{12}$ alkoxy group,
- B is a difluoroboron beta-diketone of formula (III)

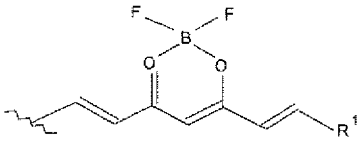 (III)

- n is 0, 1, 2, 3 or 4,
- m is 0 or 1; and

- $Q^2$ is a difluoroboron beta-diketone of formula (V)

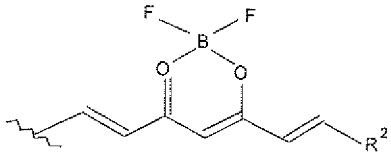 (V)

- $Q^3$ is a difluoroboron beta-diketone of formula (VI)

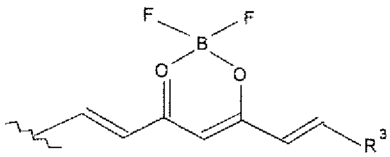 (VI)

with $R^1$, $R^2$ and $R^3$ being each independently chosen among:

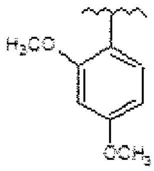 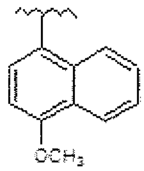 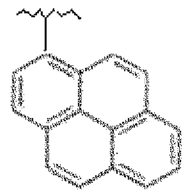 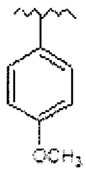

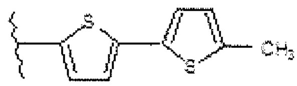 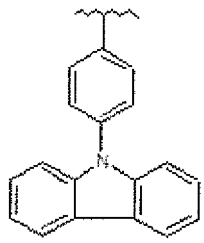 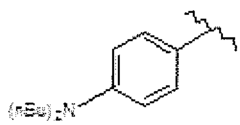

An example of the third and the fourth embodiments of the invention, is compound 3:

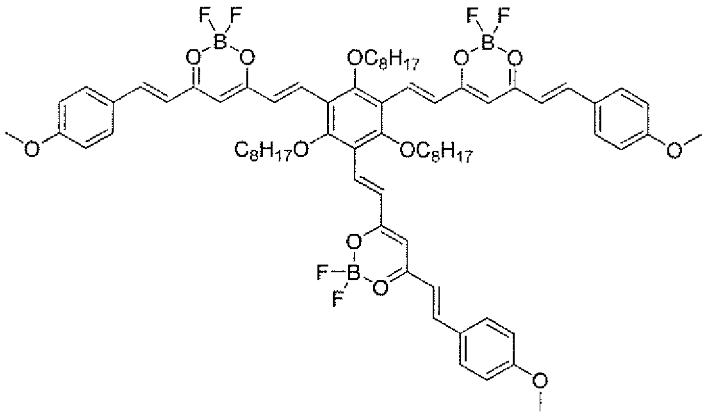

Compound 3

According to a fifth embodiment of the present invention, the compound of general formula (I) is a compound as defined above, wherein:

- $Q^1$ is represented by formula (IIb)

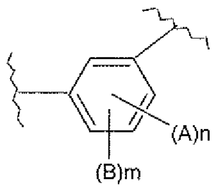

(IIb)

wherein

- $Q^1$ is substituted with $Q^2$ and $Q^3$ in meta position,
- n = 1, 2, 3 or 4,
- m=0,
- A is a $C_1$-$C_{12}$ alkoxy group; and

- $Q^2$ is a difluoroboron beta-diketone of formula (V)

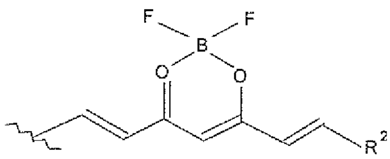

(V)

- $Q^3$ is a difluoroboron beta-diketone of formula (VI)

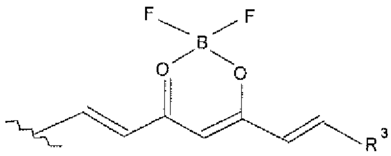

(VI)

with $R^2$ and $R^3$ being each independently chosen among:

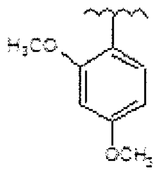
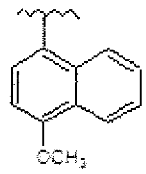
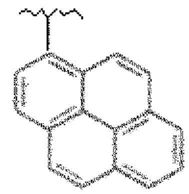
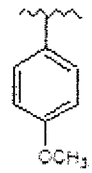

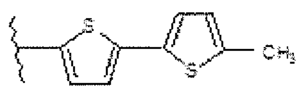
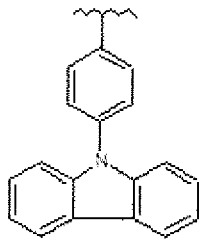
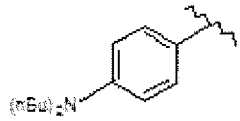

In this fifth embodiment, n is preferably equal to 3.

Examples of this fifth embodiment are compounds 4-12:

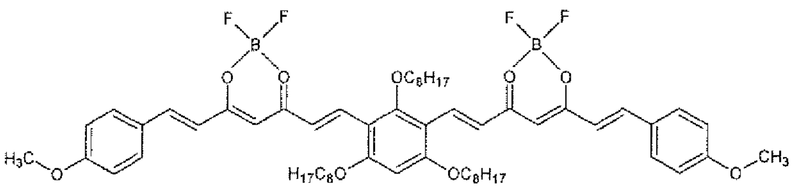

Compound 4

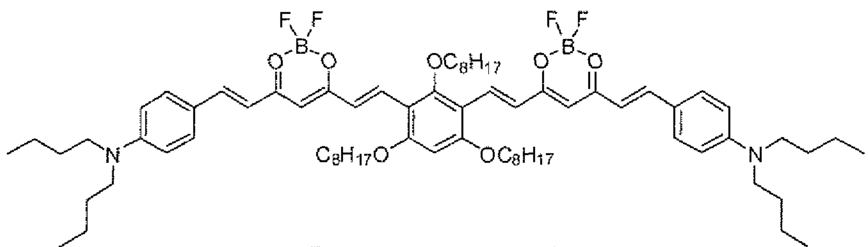

Compound 5

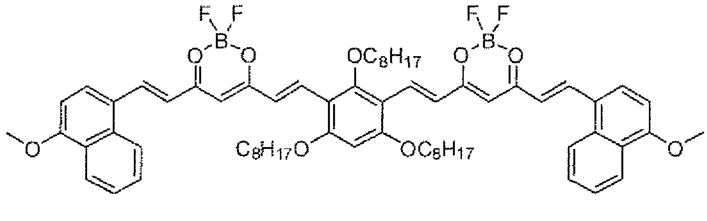

Compound 6

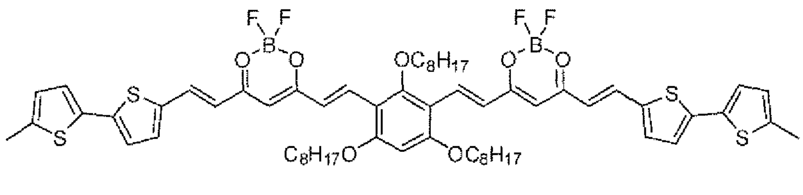

Compound 7

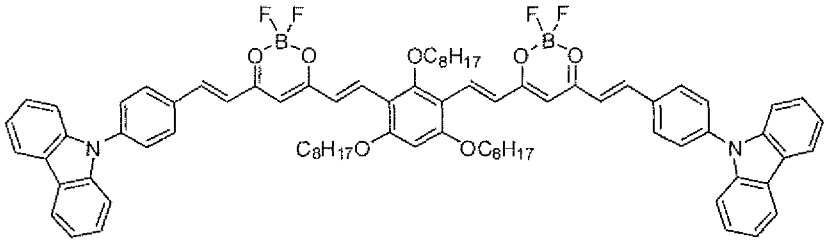

Compound 8

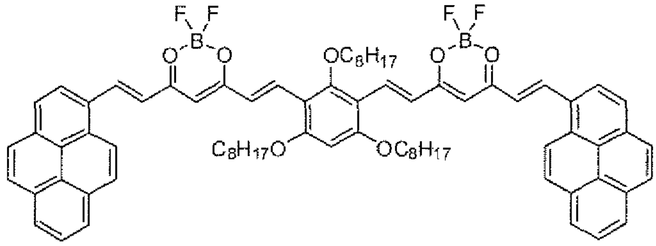

Compound 9

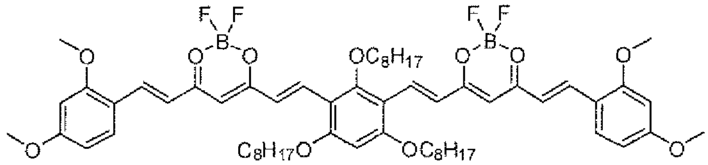

Compound 10

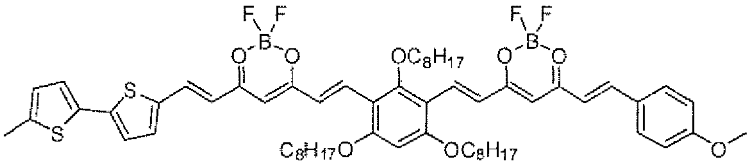

Compound 11

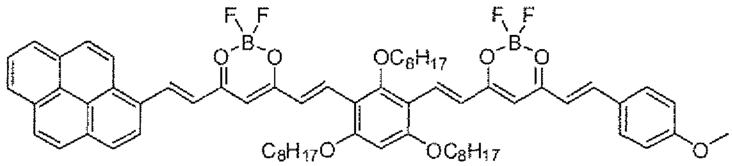

Compound 12

According to a sixth embodiment of the invention, the compound of general formula (I) is a compound as defined above, wherein:

- $Q^1$ is represented by formula (IIc)

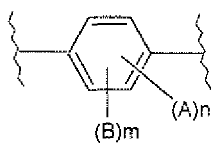

(IIc)

wherein

- $Q^1$ is substituted with $Q^2$ and $Q^3$ in para position,
- A is a $C_1$-$C_{12}$ alkoxy group,
- B is a difluoroboron beta-diketone of formula (III)

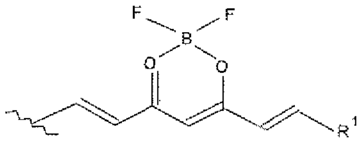 (III),

- n is 0, 1, 2, 3 or 4,
- m is 0 or 1; and

- $Q^2$ is a difluoroboron beta-diketone of formula (V)

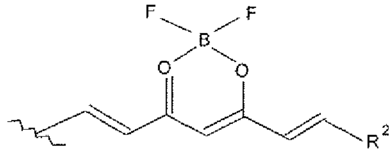 (V)

- $Q^3$ is a difluoroboron beta-diketone of formula (VI)

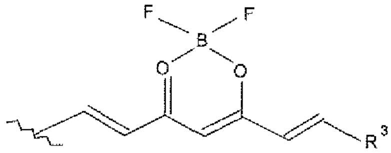 (VI)

- $R^1$, $R^2$ and $R^3$, are each independently chosen among:

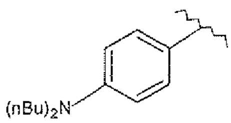, 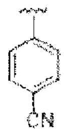, 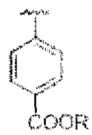, 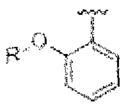, 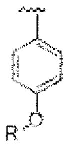, 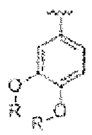, 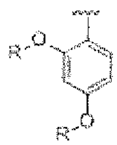, 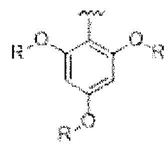,

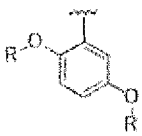, 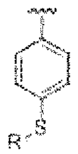, 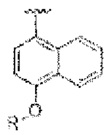, 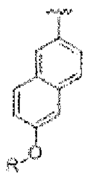, 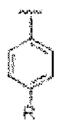, 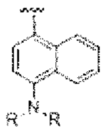, 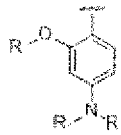, 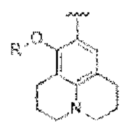, 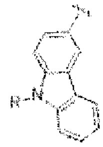,

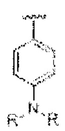, 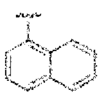, 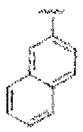, 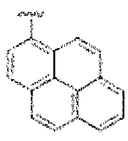, 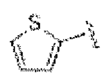, 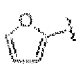, 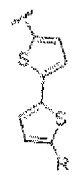, 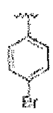, 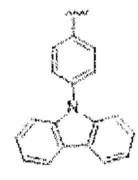,

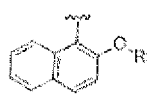, 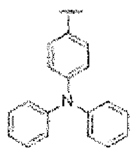

with R being each independently a hydrogen atom, a $C_1$-$C_{12}$ alkyl group.

According to a seventh embodiment of the invention, the compound of general formula (I) as defined above is a compound, wherein

- $Q^1$ is represented by formula (IIc)

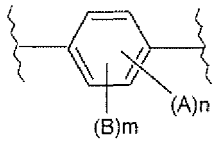

(IIc)

wherein

- $Q^1$ is substituted with $Q^2$ and $Q^3$ in para position,
- A is a $C_1$-$C_{12}$ alkoxy group,
- B is a difluoroboron beta-diketone of formula (III),

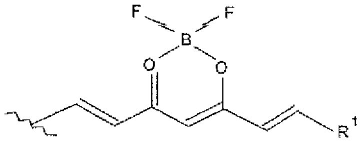

(III)

- n is 0, 1, 2, 3 or 4,
- m is 0 or 1;

- $Q^2$ is a difluoroboron beta-diketone of formula (V)

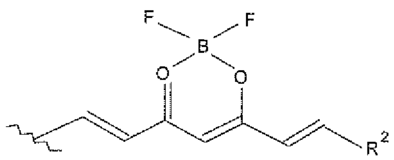

(V)

- $Q^3$ is a difluoroboron beta-diketone of formula (VI)

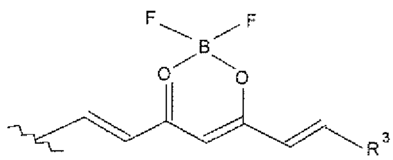

(VI)

with $R^1$, $R^2$ and $R^3$ being each independently chosen among:

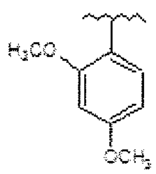 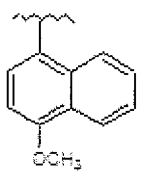 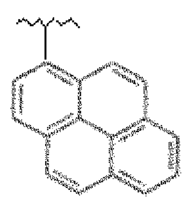 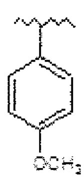

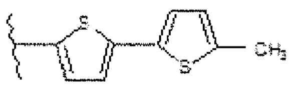
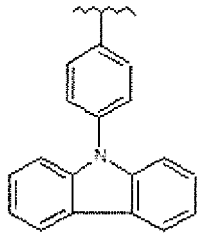
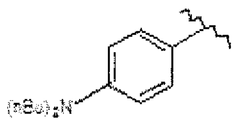

According to an eighth embodiment of the invention, the compound of general formula (I) as defined above is a compound, wherein:

- $Q^1$ is represented by formula (IIc)

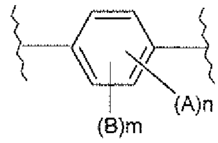

(IIc)

wherein

- $Q^1$ is substituted with $Q^2$ and $Q^3$ in para position,
- A is a $C_1$-$C_{12}$ alkoxy group,
- n is 0, 1, 2, 3 or 4,
- m=0; and

- $Q^2$ is a difluoroboron beta-diketone of formula (V)

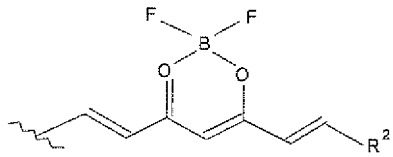

(V)

- $Q^3$ is a difluoroboron beta-diketone of formula (VI)

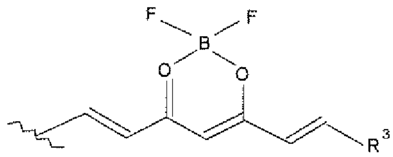

(VI)

with $R^2$ and $R^3$ being each independently chosen among:

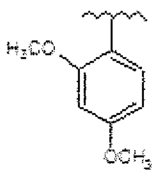
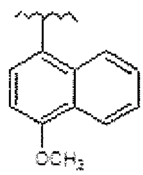
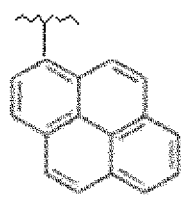
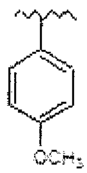

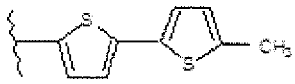

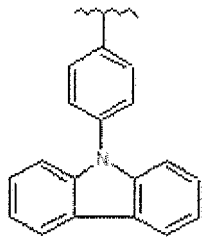

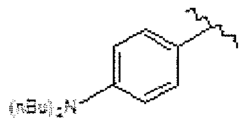

Examples of the sixth, seventh and the eighth embodiments of the invention are compounds 14-16:

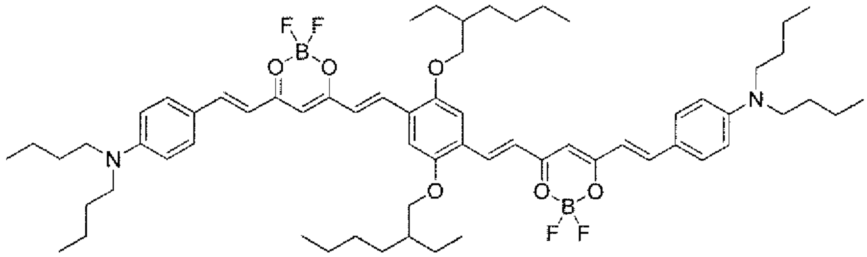

Compound 14

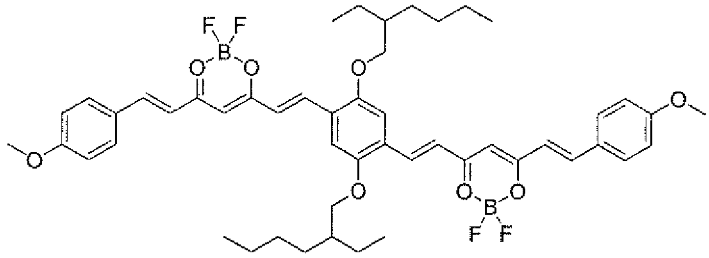

Compound 15

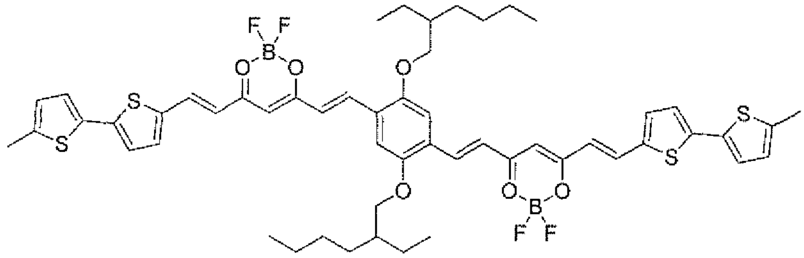

Compound 16

In embodiments one to eight, A is preferably a $C_1$-$C_8$ alkoxy.

According to a ninth embodiment of the present invention, the compound of general formula (I) as defined above is a compound, wherein:

- $Q^1$ is represented by formula (IV)

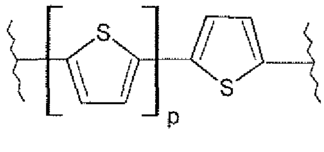

(IV)

wherein

- $Q^1$ is substituted with $Q^2$ and $Q^3$,
- p is 0, 1 or 2; and

- $Q^2$ is a difluoroboron beta-diketone of formula (V)

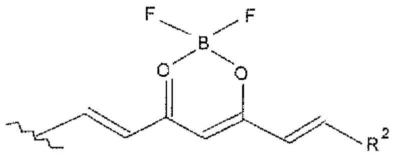

(V)

- $Q^3$ is a difluoroboron beta-diketone of formula (VI)

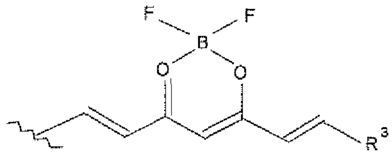

(VI)

- $R^1$, $R^2$ and $R^3$, are each independently chosen among:

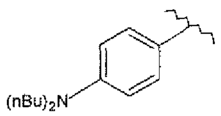, 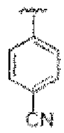, 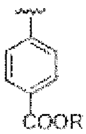, 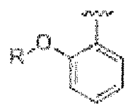, 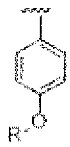, 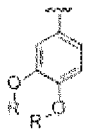, 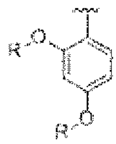, 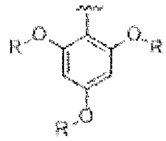,

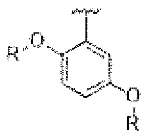, 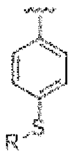, 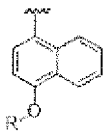, 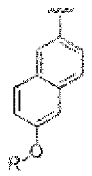, 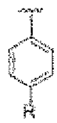, 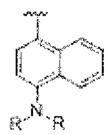, 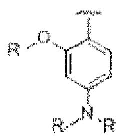, 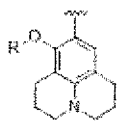, 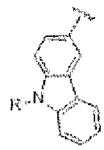,

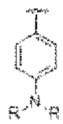, 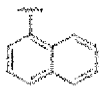, 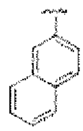, 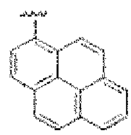, 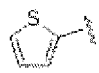, 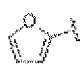, 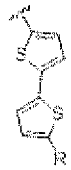, 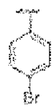, 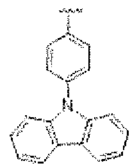,

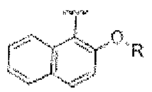, 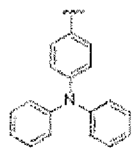

with R being each independently a hydrogen atom, a $C_1$-$C_{12}$ alkyl group.

According to a tenth embodiment of the invention, the compound of general formula (I) as defined above is a compound, wherein:

- $Q^1$ is represented by formula (IV)

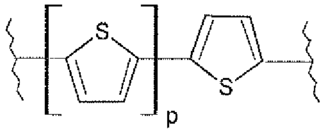

(IV)

wherein

- $Q^1$ is substituted with $Q^2$ and $Q^3$,
- p is 0, 1 or 2; and

▪ $Q^2$ is a difluoroboron beta-diketone of formula (V)

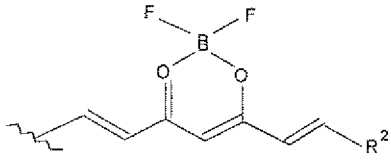

(V)

▪ $Q^3$ is a difluoroboron beta-diketone of formula (VI)

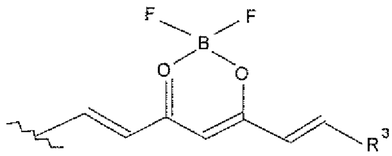

(VI)

with $R^2$ and $R^3$ being each independently chosen among:

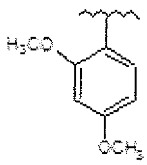
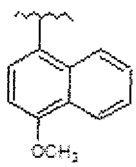
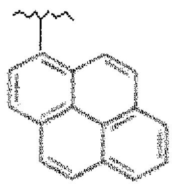
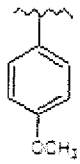

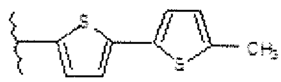
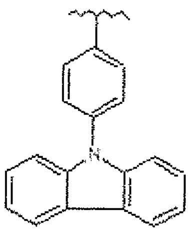
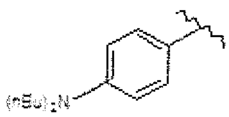

Examples of the ninth and tenth embodiments of the invention are compounds 17-21:

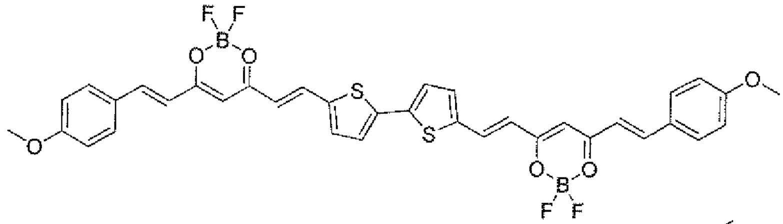

Compound 17

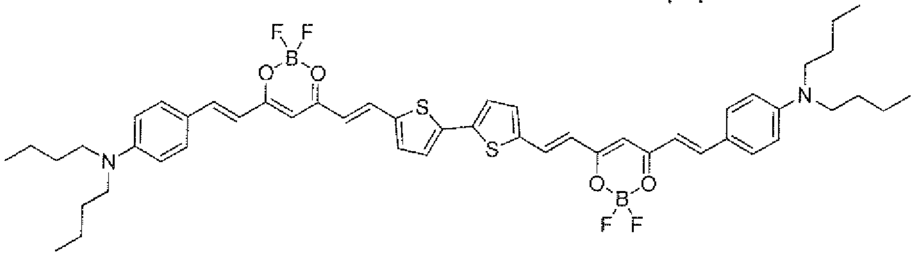

Compound 18

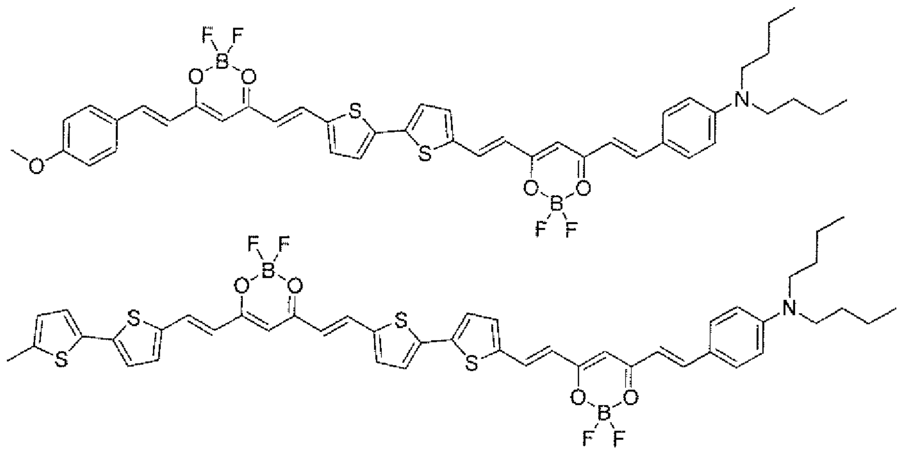

Compound 19

Compound 20

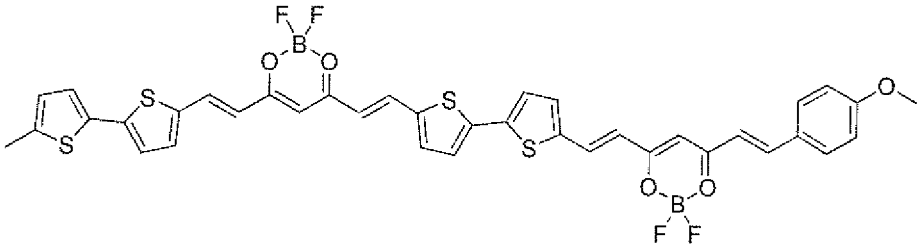

Compound 21

In the first, third, sixth, ninth embodiments, $R^1$, $R^2$ and $R^3$ are preferably independently chosen among:

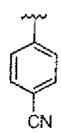 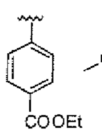 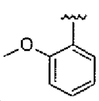 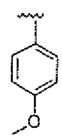 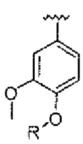 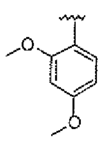 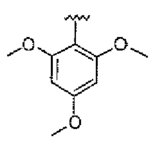 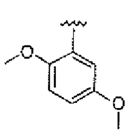 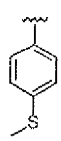

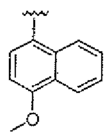 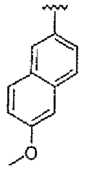 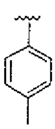 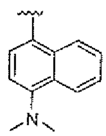 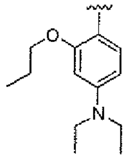 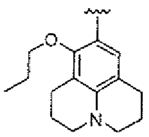 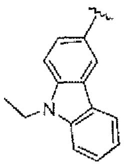 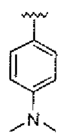

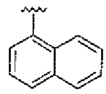 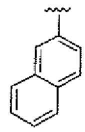 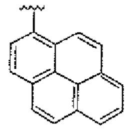 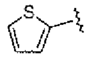 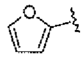 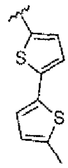 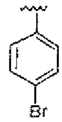 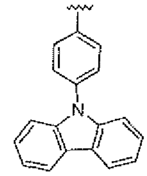

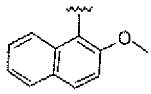 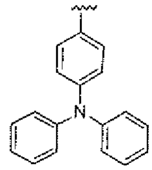

with R representing a methyl group.

In all embodiments, when R is an alkyl group, R is preferably a linear, non-substituted alkyl group.

The compounds of the invention can be obtained according to the protocol described in G. Mann, L. Beyer and A. Arrieta, Z. Chem., 1987, 27, 172-173 or in J. Med. Chem. 2006, 49, 6111-6119, as shown below:

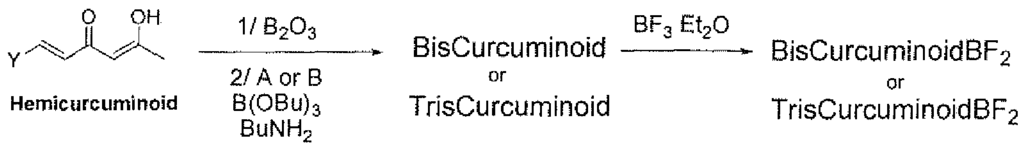


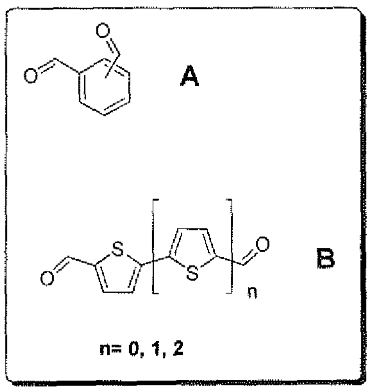


with Y chosen among:

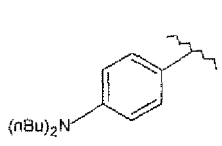, 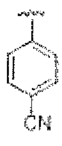, 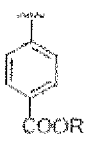, 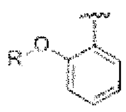, 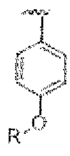, 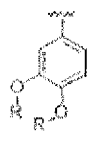, 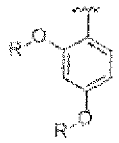, 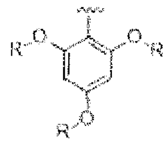,

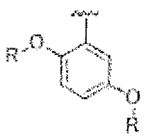, 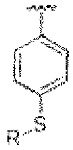, 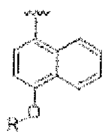, 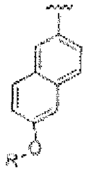, 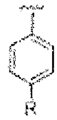, 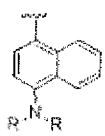, 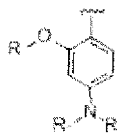, 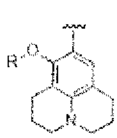, 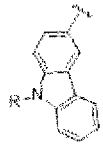,

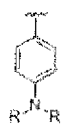, 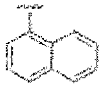, 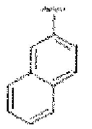, 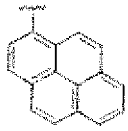, 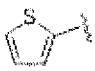, 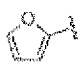, 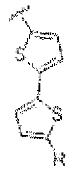, 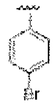, 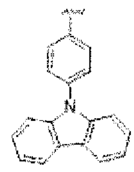,

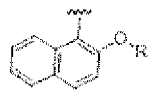, 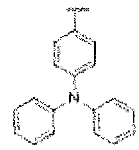.

Synthesis of Curcuminoid

In a round bottom flask, a solution of hemicurcuminoid (1 mol eq) and $B_2O_3$ (0.5 mol eq) dissolved in ethyl acetate (DMF) (10 mL) was stirred at 60 °C for 30 min. A solution of the appropriate bis-aldehyde (A or B) or tris-aldehyde (A) (1 mol eq of aldehyde function) and tri(*n*-butyl)borane (1 mol eq of aldehyde function) in ethyl acetate (10 mL) was added and the mixture was stirred for 30 min at 60 °C. A catalytic amount of *n*-butylamine (0.5 mol eq) was then added to the solution and the reaction mixture was refluxed overnight. After cooling to 60 °C, 30 mL of 0.4 M HCl were added and the mixture was stirred for 30 min. After cooling, the precipitate was filtered off and dried *in vacuo* to yield the pure BisCurcuminoid or TrisCurcuminoid-. When the solid was not pure, it was purified by column chromatography using silica gel as stationary phase.

Synthesis of Curcuminoid$BF_2$

In a round bottom flask, the ligand (1 mol eq) was solubilized in dichloromethane (20 mL) before boron trifluoride etherate (1.1 mol eq) was added. The reaction mixture was refluxed overnight. After cooling to room temperature, the solvent was evaporated and the resulting solid was suspended in diethyl ether. The precipitate was filtered off yielding the pure complex. When the crude complex was not pure, it was purified by column chromatography using silica gel as stationary phase.

The inventors have shown that compounds of the invention, display a high fluorescence quantum yield (12.5% in water with maximum emission at 692 nm and 61% in dichloromethane with maximum emission at 574 nm), and a good value of the two-photon absorption cross section (560 GM in water with excitation at 850 nm and 510 GM in dichloromethane with excitation at 800 nm), which makes them attractive fluorophores. These fluorophores have improved NIR emitting properties particularly useful in the solid state for imaging applications, for example.

Another aspect of the invention concerns the use of the compounds of formula (I) as a fluorophore. The compounds of the invention can be used both in solution, in particular in organic solvents, and in solid-state.

The present invention also concerns the use of the compounds of formula (I) in bioimaging, in particular for cells imaging; as sensors of volatile acid/base; in photodynamic therapy; in diagnosis of Alzheimer's disease; in theranostics; as optical sensors for anaerobic environment; in display and telecommunication technologies, in photovoltaics.

The compounds of the invention can represent fluorescent reporters for human beta-amyloid peptide, produced in the nerve tissues and in the blood in the course of Alzheimer's disease and may thus be used in diagnosis of Alzheimer's disease.

In the photovoltaics, the compounds of the invention may be considered as electron donors in solar cells.

Other advantages and features of the present invention may be better understood with respect to the following examples given for illustrative purposes and the accompanying figures.

Figure 1 represents the cyclic voltammogram of compound **4** in dichloromethane (DCM) solution containing 0.1M $[(^nBu_4N)PF_6]$ (Scan rate of 100 mV/s).

Figure 2 a/ Electronic absorption spectra and corrected normalized fluorescence emission spectra (conc. ≈ $10^{-6}$ M. $\lambda_{exc}$ at the absorption maximum) of compounds **X** (-, ■), **Y** (-, e), **Z** (-, ▲) and **4** (-, ▼) recorded in DCM at room temperature.

Figure 3 represents the two-photon excitation (■, higher x-coordinate and -, right y-coordinate) with their error bars, ortho-phtalaldehyde (OPA) spectra (-, lower x-coordinate and -, left y-coordinate) in DCM: a/ compound **Z** and b/ compound **4.**

Figure 4 represents the UV/visible absorption spectra of DCM solutions (-, ■) and particles in water (-, ●) and

fluorescence spectra of DCM solutions (----, □) and particles in water (----, ○) for a/ compound **X;** b/ compound **Y;** c/ compound **Z** and d/ compound **4**.

Figure 5 represents the two-photon excitation (■, higher x-coordinate and -. right y-coordinate) with their error bars, OPA spectra (-. lower x-coordinate and -. left y-coordinate) of particles of compound **4** in water,

## EXAMPLES

### Materials and Methods

### ***Spectroscopy measurements***

Spectroscopy measurements were carried out with spectroscopic grade solvents. NMR spectra ($^1H$. $^{13}C$. $^{19}F$) were recorded at room temperature on a BRUKER AC 250 operating at 400, 100, and 425 MHz for $^1H$, $^{13}C$, and $^{19}F$, respectively. Data are listed in parts per million (ppm) and are reported relative to tetramethylsilane ($^1H$ and $^{13}C$); residual solvent peaks of the deuterated solvents were used as internal standards. Mass spectra were realized in Spectropole de Marseille (http://www.spectropole.fr/). Solid state spectra and luminescence quantum yield were measured using an integrating sphere. UV/Vis-absorption spectra were measured on a Varian Cary 50. Emission spectra were measured on a Horiba-JobinYvon Fluorolog-3 spectrofluorimeter that was equipped with a three-slit double-grating excitation and a spectrograph emission monochromator with dispersions of 2.1 nm.mm$^{-1}$ (1200 grooves.mm$^{-1}$). Steady-state luminescence excitation was done using unpolarized light from a 450W xenon CW lamp and detected at an angle of 90° for dilute-solution measurements (10 mm quartz cell) and with a red-sensitive Hamamatsu R928 photomultiplier tube. Special care was taken to correct NIR-emission spectra that were obtained with the latter device. The detector was corrected according to the procedure described by Parker *et al.* (C. A. Parker, in Photoluminescence of Solutions, Elsevier Publishing, Amsterdam, 1969). The observed photomultiplier output $A_1$ was recorded at a wavelength $\lambda$, which corresponds to the apparent emission spectrum. $A_1$ is given by [Eq. (1)], where $F_1$ and $S_1$ are the corrected emission spectrum and the spectroscopic sensitivity factor of the monochromator-photomultiplier setup, respectively.

$$A_1 = (F_1)(S_1)/\lambda^2 \qquad \text{(Eq. 1)}$$

To calculate $S_1$, 4-*N,N*-dimethylamino-4'-nitrostilbene (DMANS) is used as a standard NIR fluorophore for which its corrected emission spectrum has been precisely determined (J. R. Lakowicz, Principles of Fluorescence Spectroscopy, 3rd edn., Kluwer, New-York (NY), 2006). Luminescence quantum yields

$$(\Phi_f)$$

were measured in dilute DCM solutions with an absorbance below 0.1 by using [Eq. (2)], where $OD(\lambda)$ is the absorbance at the excitation wavelength ($\lambda$), $n$ the refractive index, and $I$ the integrated luminescence intensity.

$$\Phi_{fx}/\Phi_{fr} = [OD_r(\lambda)/OD_x(\lambda)][I_x/I_r][n_x/n_r] \qquad \text{(Eq. 2)}$$

Subscripts "r" and "x" stand for reference and sample, respectively. The luminescence quantum yields were not corrected by the refractive indices. We used ruthenium trisbipyridine bischloride in water ($\Phi_{fr}$ = 0.021) as a reference for compounds that absorbed in the 450 nm region, while rhodamine B ($\Phi_{fr}$ =0.49) in EtOH was used for excitation between 540 and 560 nm.

Lifetime measurements were carried out on a HORIBA Jobin Yvon IBH FluoroLog-3 spectrofluorimeter that was adapted for time-correlated single-photon counting. For these measurements, pulsed LEDs with an appropriate wavelength were used. Emission was monitored perpendicular to the excitation pulse and spectroscopic selection was achieved by a passage through the spectrograph. A thermoelectrically cooled single-photon-detection module (HORIBA Jobin Yvon IBH, TBX-04-D) incorporating a fast-rise-time photomultiplier tube, a wide-bandwidth preamplifier, and a picosecond-constant fraction discriminator was used as the detector. Signals were acquired using an IBH DataStation Hub photon counting module and data analyses were performed by using the commercially available DAS 6 decay-analysis software package from HORIBA Jobin Yvon IBH; the reported $\tau$ values are given with an estimated uncertainty of about 10%.

Cyclic voltammetric (CV) data were acquired using a BAS 100 Potentiostat (Bioanalytical Systems) and a PC computer containing BAS100W software (v2.3). A three-electrode system with a Pt working electrode (diameter 1.6

mm), a Pt counter electrode and an Ag/AgCl (with 3M NaCl filling solution) reference electrode was used. $[(nBu)_4N]PF_6$ (0.1 M in dichloromethane) served as an inert electrolyte. Cyclic voltammograms were recorded at a scan rate of 100 $mV.s^{-1}$. Ferrocene was used as internal standard (N. G. Connelly and W. E. Geiger, Chem. Rev., 1996, 96, 877-910).

## EXAMPLE 1: Synthesis of compound 4

The synthesis of compound **4** is reported below. The two-photon excited fluorescence (TPEF) properties of **4** are characterized in solution. The preparation and fluorescence emission of organic nanoparticles obtained using **4** are also described. This approach allows giving an example of a compound as defined in the invention with improved emission ability in both the solution and the solid state.

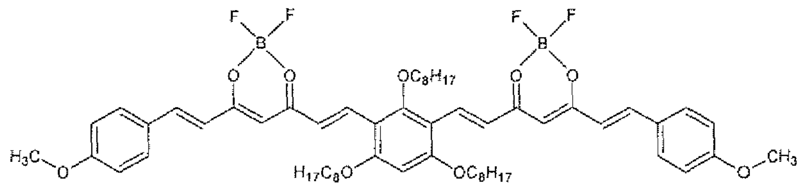

Compound 4

Synthesis of compound **4** requires first of all the preparation of compound **A.** This intermediate is prepared by a Knoevenagel reaction using an excess of acetylacetone (acac/aldehyde 3:1), providing compound **A** in a reasonable yield of 60% (G. Mann, L. Beyer and A. Arrieta, Z. Chem., 1987, 27, 172-173). Then, the reaction of two equivalents of **A** with 1,3,5-tris(*n*-octyloxy)benzene afforded **4** in a yield of 34%.

Complexation to boron difluoride is performed by reacting compound B with a slight excess of the boron trifluoride etherate in dichloromethane solution (DCM). Compound **4** is purified by crystallization or, when necessary, by column chromatography. Compound **4** is obtained as highly colored solids and characterized by $^{1}H$- and $^{19}F$-NMR spectroscopies and by high resolution mass spectrometry (HRMS).

### Synthesis of compound A ((1*E*,4*Z*)-5-hydroxy-1-(4-methoxyphenyl)hexa-1,4-dien-3-one)

In a 50 mL round bottom flask, a solution of acetylacetone (3.00 g, 30 mmol) and $B_2O_3$ (1.050 g, 15 mmol) dissolved in ethyl acetate (10 mL) was stirred at 60 °C for 30 min. A solution of anisaldehyde (1.36 g, 10 mmol) and tri(*n*-butyl)borane (2.30 g, 10 mmol) in ethyl acetate (8 mL) was added and the mixture was stirred for 30 min at 60 °C. A catalytic amount of *n*-butylamine (0.44 g, 6 mmol) was then added to the solution and the reaction mixture was refluxed overnight. After cooling to 60 °C, 30 mL of 0.4 M HCl were added and the mixture was stirred for 30 min. After cooling, the precipitate was filtered off. The residual solution was evaporated. The oily compound was dissolved in dichloromethane. The organic layer was washed with water, brine, dried over $MgSO_4$ and evaporated to dryness. The oily crude was purified by column chromatography on silica using a mixture of cyclohexane and dichloromethane (gradient from 1/1 to 3/1) yielding the pure **A** as a yellowish solid (1.20 g, 55%).

### Synthesis of compound B ((1*E*,1'*E*,4*Z*,4'*Z*,6*E*,6'*E*)-1,1'-(2,4,6-tris(octyloxy)-1,3-phenylene)bis(5-hydroxy-7-(4-methoxyphenyl)hepta-1,4,6-trien-3-one))

In a 50 mL round bottom flask, a solution of A (1 mol eq) and $B_2O_3$ (0.5 mol eq) dissolved in ethyl acetate (10 mL) was stirred at 60 °C for 30 min. A solution of the appropriate aldehyde (1 mol eq of aldehyde function) and tri(*n*-butyl)borane (1 mol eq of aldehyde function) in ethyl acetate (10 mL) was added and the mixture was stirred for 30 min at 60 °C. A catalytic amount of *n*-butylamine (0.5 mol eq) was then added to the solution and the reaction mixture was refluxed overnight. After cooling to 60 °C, 30 mL of 0.4 M HCl were added and the mixture and stirred for 30 min. After cooling, the precipitate was filtered off and dried *in vacuo* to yield the pure compound **B.**

Compound **B**: ((1*E*,1'*E*,4*Z*,4'Z,6*E*,6'*E*)-1,1'-(2,4,6-tris(octyloxy)-1,3-phenylene)bis(5-hydroxy-7-(4-methoxyphenyl)hepta-1,4,6-trien-3-one))

Dark orange solid; yield: 34%; $^{1}H$ NMR (400 MHz, $CDCl_3$, ppm) : $\delta$ = 7.92 (d, $^{3}J$ = 16.1 Hz, 2H), 7.60 (d, $^{3}J$ = 15.8 Hz, 2H), 7.49 (d, $^{3}J$ = 8.7 Hz, 4H), 7.04 (d, $^{3}J$ = 16.1 Hz, 2H), 6.89 (d, $^{3}J$ = 8.7 Hz, 4H), 6.48 (d, $^{3}J$ = 15.8 Hz, 2H), 6.23 (s, 1H), 5.69 (s, 2H), 4.05 (t, $^{3}J$ = 6.2 Hz, 4H), 3.83 (m, 6H), 3.77 (t, $^{3}J$ = 6.3 Hz, 4H), 1.88 (m, 6H), 1.53 (m, 6H), 1.30 (m, 24H), 0.87 (m, 9H); $^{13}C$ NMR (100 MHz, $CDCl_3$): $\delta$ = 184.09, 183.78, 161.66, 161.23, 139.80, 131.86, 129.78, 128.00, 125.68, 122.29, 114.41, 111.44, 101.76, 92.63, 69.04, 55.47, 31.97, 29.68, 29.53, 29.44, 29.17, 26.42, 26.36, 22.81, 14.23. HRMS (ESI+) $[M + 2H]^{2+}$ calcd for $C_{58}H_{80}O_8^{+}$ m/z= 460.2901, found m/z= 460.2903.

## General procedure for borondifluoride complexes

In a 50 mL round bottom flask, the ligand (1 mol eq) was solubilized in dichloromethane (20 mL) before boron trifluoride etherate (1.1 mol eq) was added. The reaction mixture was refluxed overnight. After cooling to room temperature, the solvent was evaporated and the resulting solid was suspended in diethyl ether. The precipitate was filtered off yielding the pure compound **4.**

Compound **4:** ((1*E*,1'*E*,4*Z*,4'*Z*,6*E*,6'*E*)-1,1'-(2,4,6-tris(octyloxy)-1,3-phenylene)bis(5-(difluoroboryloxy)-7-(4-methoxyphenyl)hepta-1,4,6-trien-3-one))

Red solid; yield: 97%; $^1$H NMR (400 MHz, DMSO, ppm) : $\delta$ = 8.25 (d, $^3J$ = 16 Hz, 2H), 7.97 (d, $^3J$ = 15.5 Hz, 2H), 7.55 (d, $^3J$ = 8.5 Hz, 4H), 7.14 (d, $^3J$ = 16 Hz, 2H), 6.93 (d, $^3J$ = 8.5 Hz, 4H), 6.54 (d, $^3J$ = 15.5 Hz, 2H), 6.26 (s, 1H), 5.56 (s, 1H), 4.14 (t, $^3J$ = 6.5 Hz, 4H), 3.85 (s, 6H), 3.80 (t, $^3J$ = 6.5 Hz, 2H), 1.92 (m, 6H), 1.32 (m, 30H), 0.90 ppm (m, 9H); $^{19}$F NMR (235 MHz, DMSO): $\delta$ = -140.91 ($^{10}$B-F, 0.2), -140.97 ppm ($^{11}$B-F, 0.8). HRMS (ESI+) [M + 2Na]$^{2+}$ calcd for $C_{58}H_{76}O_9B_2F_4Na_2^{2+}$ m/z= 530.2707, found m/z= 530.2705.

## EXAMPLE 2: Electrochimical, photophysical and solid-date optical properties of compound 4 and comparison with other compounds.

It is reported herein a study of electrochemical and optical properties in organic solvents of four compounds: three borondifluoride complex of a mono-curcuminoid compounds (named X, Y and Z) versus compound 4, a borondifluoride complex of a biscurcuminoid system. The two-photon excited fluorescence (TPEF) properties of the four compounds were characterized in solution.

Molecular structures of the borondifluoride curcuminoid derivatives X, Y, Z are presented below.

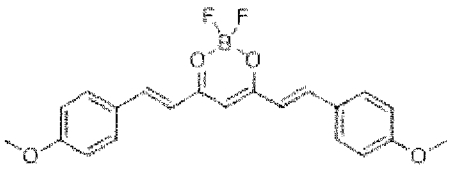
X

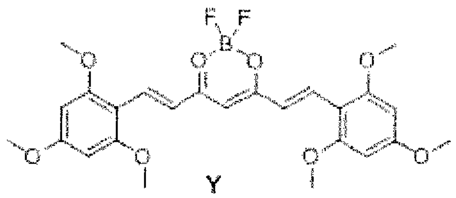
Y

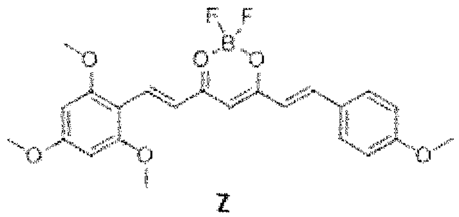
Z

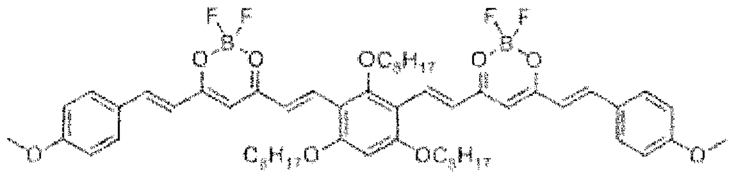
4

### 2.1. Comparison of electrochemical properties

The electrochemical properties of compounds X, Y, Z and 4 were investigated in dicholoromethane (DCM) solution containing 0.1 M of ($n$-Bu)$_4$NPF$_6$.

The cyclic voltammograms (CV) are given in Figure 1 and the oxidation and reduction half-wave potential values ($E_{1/2}$ *vs.* ferrocene/ferrocenium) are collected in Table 1.

Table 1. Oxidation and reduction half-wave potential values for the studied dioxaborine derivatives (10µM) in dichloromethane solution vs. Fc/Fc+● using tetrabutylammonium hexafluorophosphate as electrolyte (100µM), the values are given in volt. (a) Electrochemical HOMO-LUMO gap ΔEg= (E1/2ox)1 - E1/2red, (b) The second oxidation process occurs at potentials out of the solvent electrochemical window.

| Compound | E1/2red | (E1/2ox)1 | (E1/2ox)2 | ΔEg |
|---|---|---|---|---|
| X | -1.27 | 1.10 | -b | 2.37 |
| Y | -1.44 | 0.72 | 1.09 | 2.16 |
| Z | -1.37 | 0.89 | 1.31 | 2.26 |

(continued)

| Compound | E1/2red | (E1/2ox)1 | (E1/2ox)2 | ∆Eg |
|---|---|---|---|---|
| 4 | -1.30 | 1.05 | -b | 2.35 |

The increase of the oxidation potential in **4** relative to Z could be due to presence the second curcuminoid moiety. In addition, since both reduction and oxidation processes of 4 involve the same number of electrons, it may be assumed that methoxyphenyl end-groups and dioxaborine rings in 4 are simultaneously oxidized and reduced, respectively, at the same potential.

**2.2. Comparison of photophysical properties**

The electronic absorption spectra of compounds X, Y, Z and 4 were recorded in DCM solutions (Figure 2) and the spectroscopic data are reported in Table 2. The spectra consist mainly of one intense transition band at low energy (450 - 550 nm) attributed to a strongly allowed $\pi$-$\pi^*$ transition. A second electronic transition band of much lower intensity appears as a shoulder at higher energy (< 400 nm, *vide infra*). The spectra of compounds X, Y and Z display identical shape of the absorption profiles, they only differ by the position of the bands. In contrast, compound 4 exhibits a low-energy absorption band with very different shape, full width at half maximum, and intensity. The molar absorption coefficient determined for 4 is twice as high as that of compounds X, Y and Z. The more complex shape of the absorption band is likely to stem from intramolecular exciton coupling between the two curcuminoid chromophores.

Compounds X, Y, Z and 4 are fluorescent in the visible region (540 - 575 nm) upon excitation into the low-energy transition band and exhibit fluorescence quantum yields ranging from 44 to 61 % in DCM. In agreement with electronic absorption data, an increase of the donor strength causes a red-shift of the fluorescence emission from 538 nm (X) to 574 nm (4). It is worth noting that the highest value of $\Phi_f$ (61 %) is obtained for complex 4, giving a high brightness value of *ca.* 87000 $M^{-1}$ $cm^{-1}$.

Table 2. Spectroscopic data and photophysical properties of all borondifluoride compounds solvated in DCM and as particles in water at room temperature[a]

| compound | UV-vis | | Fluorescence | | | | | | | TPEF | | |
|---|---|---|---|---|---|---|---|---|---|---|---|---|
| | $\lambda^{abs}$ | $\varepsilon_{max}$ | $\lambda^{cm}$ | $\Delta v_{ST}$ | $\Phi_f$ | $B^1$ | $\tau_f$ | $k_f$ | $k_m$ | $\lambda^2_{max}$ | $\sigma^{TPA}$ | $B^2$ |
| **X** (DCM) | 488 | 75480 | 538 | 1904 | 0.44 | 33211 | 1.30 | 3.4 | 4.3 | 770 | 155 | 68 |
| **Y** (DCM) | 524 | 84860 | 566 | 1416 | 0.52 | 44127 | 1.72 | 3.0 | 2.8 | 810 | 248 | 129 |
| **Z** (DCM) | 511 | 74510 | 561 | 1957 | 0.46 | 34275 | 1.74 | 2.6 | 3.1 | 790 | 208 | 96 |
| **4** (DCM) | 529 | 143140 | 574 | 1421 | 0.61 | 87315 | 1.69 | 3.6 | 2.3 | 800 | 513 | 313 |
| **X** (water) | 451 | 34170 | 710 | 8088[b] | 0.06 | 2050 | 6.44 | 0.09 | 1.5 | 780 | 210 | 13 |
| **Y** (water) | 489 | 27380 | 717 | 6503[b] | 0.035 | 783 | 1.32<br>2.91[c] | - | - | -[d] | -[d] | -[d] |
| **Z** (water) | 407 | 29500 | 711 | 10505[b] | 0.015 | 443 | 2.13<br>6.01[c] | - | - | -[d] | -[d] | -[d] |
| **4** (water) | 468 | 55620 | 692 | 6917[b] | 0.125 | 6952 | 1.89<br>6.64[c] | - | - | 850 | 560 | 70 |

[a] Absorption maximum wavelengths $\lambda^{abs}$ (nm); Molar absorption coefficients at maximum $\varepsilon_{max}$ ($M^{-1}$ $cm^{-1}$); Fluorescence maximum wavelengths $\lambda^{em}$ (nm); Stokes shifts $\Delta v_{ST}$ ($cm^{-1}$); Fluorescence quantum yields $\Phi_f$; Brightness $B = \Phi_f x\ \varepsilon$($M^{-1}$ $cm^{-1}$); Fluorescence lifetimes $\tau_f$ (ns); Radiative $k_f$ ($10^8$ $s^{-1}$) and nonradiative $k_{nr} = (1 - \Phi_f)/\tau_f$ ($10^8$ $s^{-1}$) rate constants; Two-photon absorption maximum $\lambda^2_{max}$ (nm); Two-photon cross section $\sigma^{TPA}$ (GM); Two-photon brightness $B^2 = \Phi_f$ x $\sigma^{TPA}$ (GM). [b] Pseudo-Stokes shift determined using the maximum absorption. [c] A biexponential decay was found [d] Not determined due to high scattering of light with those particles.

Two-photon excited fluorescence emission and excitation spectra of X, Y , Z and 4 were recorded in the 700-1000 nm wavelength range using a femtosecond Ti-Sapphire pulsed laser source, according to the experimental protocol described by Webb *et al.* (C. Xu and W. W. Webb, J. Opt. Soc. Am. B, 1996, 13, 481-491) using coumarin-307 and rhodamine B as references (C. Xu and W. W. Webb, J. Opt. Soc. Am. B, 1996, 13, 481-491). The observation of a quadratic dependence of the fluorescence intensity *versus* incident laser power at several wavelengths unambiguously confirmed that the origin of the fluorescence emission can be assigned to a TPA process in DCM solution. In the experimental laser power range used for these measurements, it was checked that no saturation or photodegradation occurred. The two-photon excitation spectra of X, Y, Z and 4 in DCM are shown in Figure 3 and the corresponding data are reported in Table 2. X has a TPA cross section ($\sigma^{TPA}$) of 155 GM at 770 nm while compound Y, containing stronger donating groups, has its maximum red-shifted to 810 nm with a larger $\sigma^{TPA}$ value of *ca.* 248 GM. It is interesting to note that Z presents an intermediate $\sigma^{TPA}$ value of 208 GM at 790 nm. For compound 4, as already observed for the molar absorption coefficient, the two-photon cross section is slightly more than twice (*i.e.* 513 GM) the value determined for Z. This gives a two-photon brightness of 313 GM, that is much higher than those obtained with the model borondifluoride complexes X, Y, Z.

## 2.3. Comparison of solid-state optical properties

Solid-state particles were prepared by quickly adding a concentrated THF solution of the compound Y, Z and 4 into water according to the classical fast precipitation method (H. Kawai, H. S. Nalwa, H. Oikawa, S. Okada, H. Matsuda, N. Minami, A. Kakuda, K. Ono, A. Mukoh and H. Nakanishi, Jpn. J. Appl. Phys., 1992, 31, L1132-L1134). The so-obtained suspensions enabled the measurement of the UV/visible absorption and fluorescence spectra of the aggregated molecules. The preparation of X has been reported by Felouat *et al.* (A. Felouat, A. D'Aléo and F. Fages, J. Org. Chem., 2013, 78, 4446-4455) and the resulting spectroscopic data are recalled here for the sake of comparison. The shape of the electronic absorption spectra of compounds X, Y Z, and 4 is strongly affected relative to the solution spectra. The absorption profiles of X, Y and 4 (Figures 4a, 4b, and 4d, respectively) are complex, broad, and their maximum presents a hypsochromic shift (*ca.* 30 - 60 nm). These observations show that excitonic interactions prevail in the condensed phase, which could be correlated with single-crystal X-ray diffraction data in the case of X. The absorption spectrum of Z displays a very different effect (Figures 4c). The low-energy transition is considerably blue-shifted (of *ca.* 96 nm) and has a narrower shape as compared to that of the previous curcuminoid particles. These spectroscopic features are the signature of H-aggregation in the solid state for compound Z. Noticeably, 4, also containing unsymmetrical curcuminoid chromophores, does not adopt such arrangement.

Particles of compounds Y, Z and 4 fluoresce in the NIR, from 692 to 717 nm (Figure 4, Table 2). The spectra are considerably red-shifted (superior to 95 nm) as compared to those obtained in solution in the highly polar acetonitrile solvent. Such long emission wavelengths can be attributed to chromophore packing in the solid state, as shown by X-ray diffraction for X and by electronic absorption spectroscopy for the others (Figure 4). The fluorescence quantum yields of X, Y and Z are found in the range of 1.5 - 6.0 % which are significant values for aggregated organic dyes emitting in the NIR. Not surprisingly, the H-aggregated compound Z affords the lowest value of 1.5 %. Remarkably, the $\Phi_f$ of dye 4 reaches a substantial value of 12.5 % in the solid state, which makes compound 4 a very bright solid-state NIR fluorophore (brightness at 700 nm of 6952 $M^{-1}cm^{-1}$). Actually, a rather large part of emitted photons by the four compounds have energies below 700 nm because the particle emission spectra are broad. However, it can be highlighted that, when only photons at longer wavelength than 700 nm are integrated, dye 4 remains the most luminescent borondifluoride complex of the series with a NIR luminescence quantum yield of *ca.* 6.5% while the other three dyes have quantum yields of 4.0, 1.5 and 1.0 %, respectively.

In addition, two-photon properties of 4 could be measured and compared to the previously reported X particles. Noticeably, the Y and Z particles could not be measured due to the much higher light scattering in those samples which precluded obtaining reliable data. As observed in DCM solution and for X in water, the two-photon maximum of 4 does not overlap the maximum of the one photon absorption $S_0$-$S_1$ transition (Figure 5) but it better matches the $S_0$-$S_2$ one (*vide supra*). This maximum is located at 850 nm with a two-photon cross section of *ca.* 560 GM. Such two-photon cross section value is 2.5 times higher than that of X which, associated to the higher fluorescence quantum yield of 4, results in a much higher two-photon brightness for 4 (more than 5 times greater than the one of X).

## 2.4. Conclusion

In compound 4, the absorption spectrum reveals that an intramolecular excitonic interaction exists in solution, showing that the two chromophoric units are not optically independent. Compound 4 displays a high fluorescence quantum yield, and a good value of the two-photon absorption cross section, which makes it an attractive fluorophore. Like compounds X, Y and Z, compound 4 experiences intermolecular interactions in the condensed phase. However, it exhibits the higher value of fluorescence quantum yield within the series investigated.

## Claims

**1.** A compound of general formula (I):

$Q^2$-$Q^1$-$Q^3$ (I)

wherein

- $Q^1$ represented by formula (II)

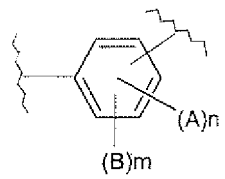

(II)

wherein

- $Q^1$ is substituted with $Q^2$ and $Q^3$,
- A is a $C_1$-$C_{12}$ alkoxy group,
- B is a difluoroboron beta-diketone of formula (III)

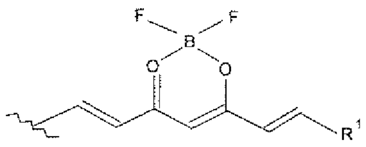

(III)

- n is 0, 1, 2, 3 or 4,
- m is 0 or 1; or

- $Q^1$ is represented by formula (IV)

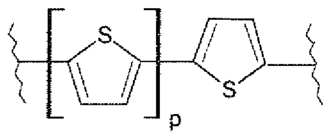

(IV)

wherein

- $Q^1$ is substituted with $Q^2$ and $Q^3$,
- p is 0, 1 or 2;

- $Q^2$ is a difluoroboron beta-diketone of formula (V)

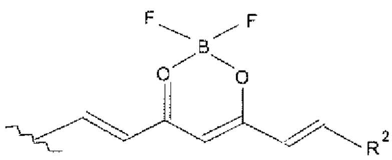

(V)

- $Q^3$ is a difluoroboron beta-diketone of formula (VI)

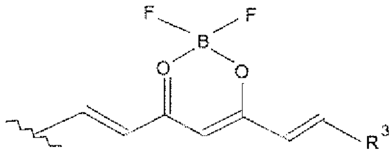 (VI)

- $R^1$, $R^2$ and $R^3$, are each independently chosen among:

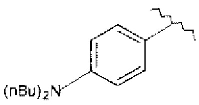, 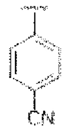, 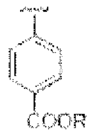, 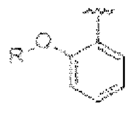, 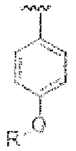, 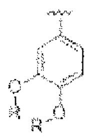, 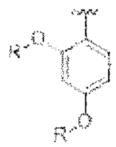, 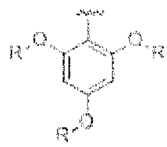,

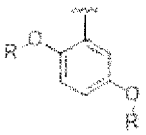, 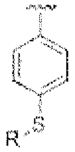, 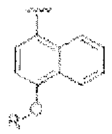, 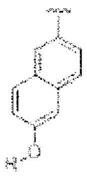, 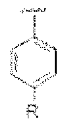, 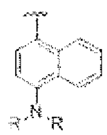, 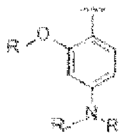, 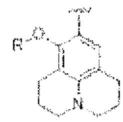, 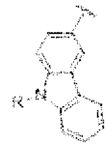,

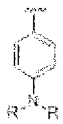, 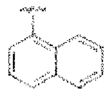, 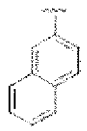, 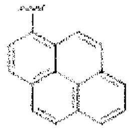, 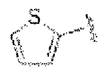, 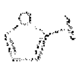, 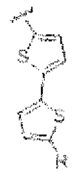, 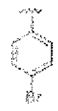, 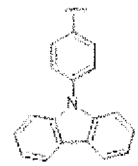,

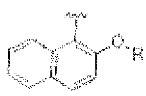, 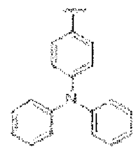

with R being each independently a hydrogen atom, a $C_1$-$C_{12}$ alkyl group.

**2.** The compound of formula (I) according to claim 1, wherein

- $Q^1$ is represented by formula (IIa)

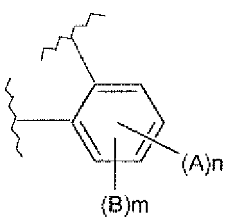 (IIa)

wherein

- $Q^1$ is substituted with $Q^2$ and $Q^3$ in ortho position,
- A is a $C_1$-$C_{12}$ alkoxy group,
- B is a difluoroboron beta-diketone of formula (III),

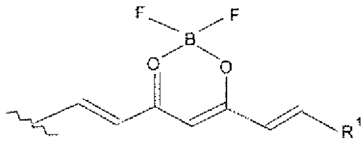
(III)

- n is 0, 1, 2, 3 or 4,
- m is 0 or 1; and

- $Q^2$, $Q^3$, R, $R^1$, $R^2$ and $R^3$, are as defined in claim 1.

**3.** The compound of formula (I) according to claim 1 or 2, wherein

- $Q^1$ is represented by formula (IIa)

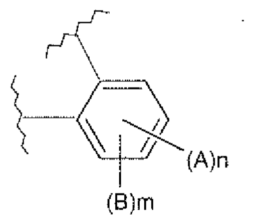
(IIa)

wherein

- $Q^1$ is substituted with $Q^2$ and $Q^3$ in ortho position,
- n=m=0; and

- $Q^2$ and $Q^3$ are as defined in claim 1, with $R^2$ and $R^3$ being each independently chosen among:

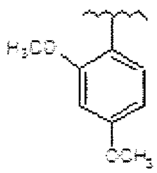
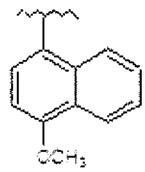
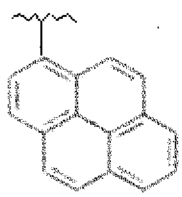
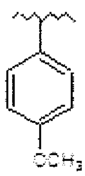

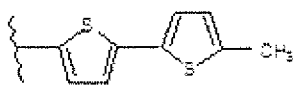
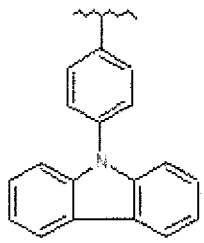
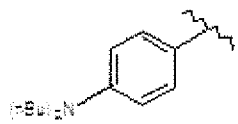

**4.** The compound of formula (I) according to claim 1, wherein

- $Q^1$ is represented by formula (IIb)

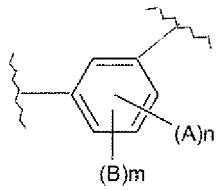

(IIb)

wherein

- $Q^1$ is substituted with $Q^2$ and $Q^3$ in meta position,
- A is a $C_1$-$C_{12}$ alkoxy group,
- B is a difluoroboron beta-diketone of formula (III)

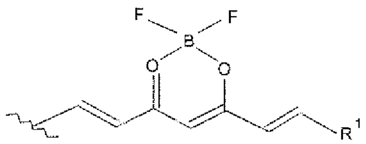

(III)

- n is 0, 1, 2, 3 or 4,
- m is 0 or 1; and

- $Q^2$, $Q^3$, R, $R^1$, $R^2$ and $R^3$ are as defined in claim 1.

**5.** The compound of formula (I) according to claim 1 or 4, wherein

- $Q^1$ is represented by formula (IIb)

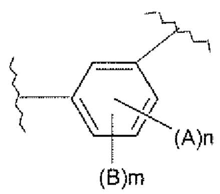

(IIb)

wherein

- $Q^1$ is substituted with $Q^2$ and $Q^3$ in meta position,
- A is a $C_1$-$C_{12}$ alkoxy group,
- B is a difluoroboron beta-diketone of formula (III)

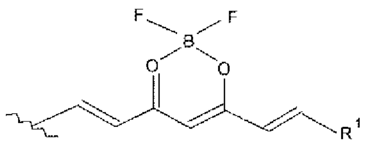

(III)

- n is 0, 1, 2, 3 or 4,
- m is0 or 1; and

- $Q^2$ and $Q^3$ are as defined in claim 1, with $R^1$, $R^2$ and $R^3$ being each independently chosen among:

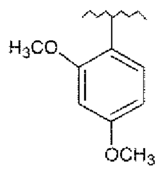 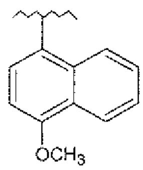 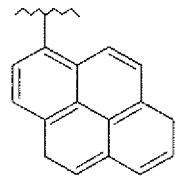 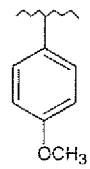

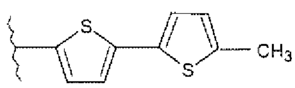 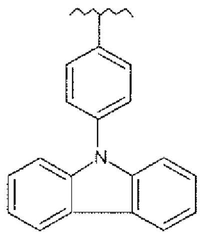 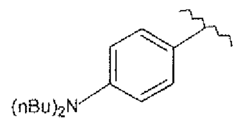 .

6. The compound of formula (I) according to anyone of claims 1, 4 and 5, wherein

- $Q^1$ is represented by formula (IIb)

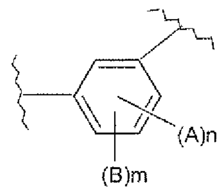

(IIb)

wherein

- $Q^1$ is substituted with $Q^2$ and $Q^3$ in meta position,
- n = 1, 2, 3 or 4,
- m=0,
- A is a $C_1$-$C_{12}$ alkoxy group; and

- $Q^2$ and $Q^3$ are as defined in claim 1, with $R^2$ and $R^3$ being each independently chosen among:

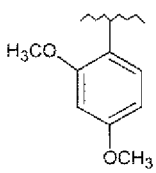 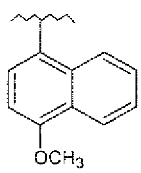 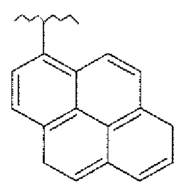 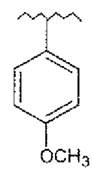

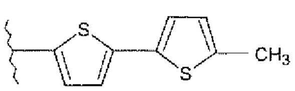 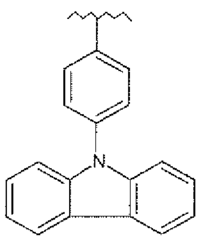 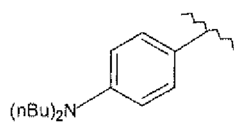 .

7. The compound of formula (I) according to claim 1, wherein

- $Q^1$ is represented by formula (IIc)

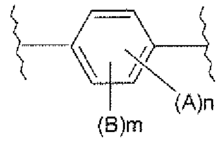 (IIc)

wherein

- $Q^1$ is substituted with $Q^2$ and $Q^3$ in para position,
- A is a $C_1$-$C_{12}$ alkoxy group,
- B is a difluoroboron beta-diketone of formula (III)

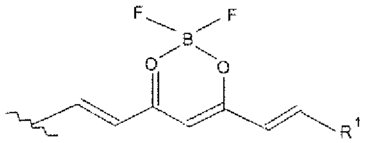 (III),

- n is 0, 1, 2, 3 or 4,
- m is 0 or 1; and

▪ $Q^2$, $Q^3$, R, $R^1$, $R^2$ and $R^3$ are as defined in claim 1.

**8.** The compound of formula (I) according to claim 1 or 7, wherein

▪ $Q^1$ is represented by formula (IIc)

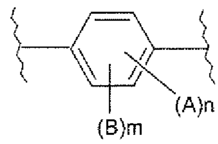 (IIc)

wherein

- $Q^1$ is substituted with $Q^2$ and $Q^3$ in para position,
- A is a $C_1$-$C_{12}$ alkoxy group,
- B is a difluoroboron beta-diketone of formula (III),

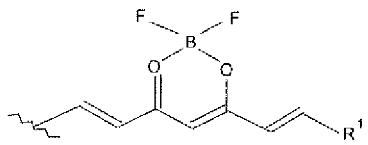 (III)

- n is 0, 1, 2, 3 or 4,
- m is 0 or 1;

▪ $Q^2$ and $Q^3$ are as defined in claim 1, with $R^1$, $R^2$ and $R^3$ being each independently

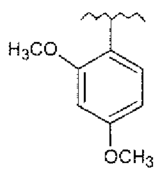 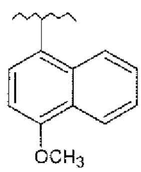 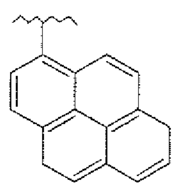 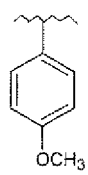

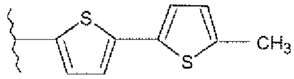 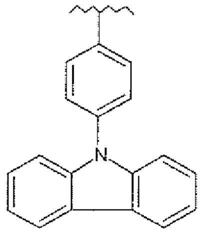 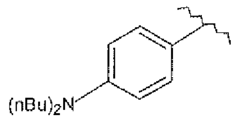

9. The compound of formula (I) according to any one of claims 1, 7 and 8, wherein

- $Q^1$ is represented by formula (IIc)

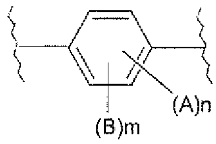

(IIc)

wherein

- $Q^1$ is substituted with $Q^2$ and $Q^3$ in para position,
- A is a $C_1$-$C_{12}$ alkoxy group,
- n is 0, 1, 2, 3 or 4,
- m=0; and

- $Q^2$ and $Q^3$ are as defined in claim 1, with $R^2$ and $R^3$ being each independently chosen among:

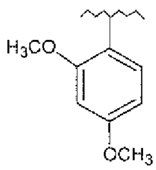 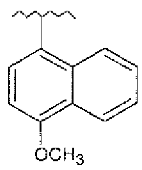 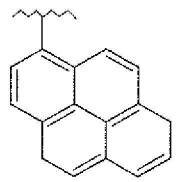 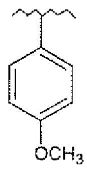

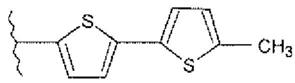 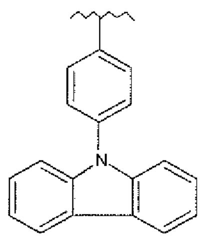 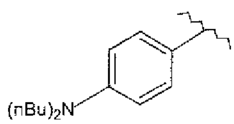

10. The compound of formula (I) according to claim 1, wherein

- $Q^1$ is represented by formula (IV)

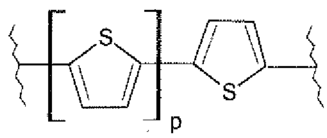

(IV)

wherein

- $Q^1$ is substituted with $Q^2$ and $Q^3$,

- p is 0, 1 or 2; and

▪ $Q^2$, $Q^3$, $R^2$ and $R^3$ are as defined in claim 1.

**11.** The compound of formula (I) according to claim 1 or 10, wherein

▪ $Q^1$ is represented by formula (IV)

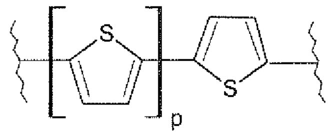

(IV)

wherein

- $Q^1$ is substituted with $Q^2$ and $Q^3$,
- p is 0, 1 or 2; and

▪ $Q^2$ and $Q^3$ are as defined in claim 1, with $R^2$ and $R^3$ being each independently chosen among:

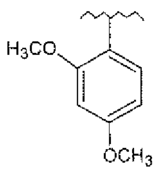
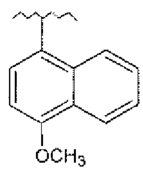
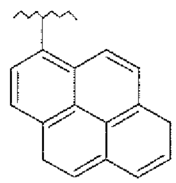
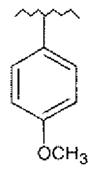
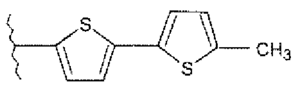
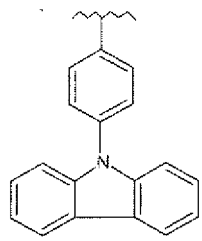
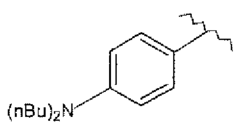

**12.** Use of a compound of formula (I) according to any one of claims 1 to 11, as a fluorophore.

**13.** A compound of formula (I) according to any one of claims 1 to 11, for use in bioimaging, as sensors of volatile acid/base, in photodynamic therapy, in diagnosis of Alzheimer's disease, in theranostics, as optical sensors for anaerobic environment, in display and telecommunication technologies, in photovoltaics.

## Patentansprüche

**1.** Verbindung der allgemeinen Formel (I):

$$Q^2\text{-}Q^1\text{-}Q^3 \qquad (I)$$

wobei

▪ Q1 der Formel (II) entspricht:

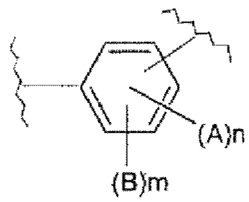

(II)

wobei

- $Q^1$ mit $Q^2$ und $Q^3$ substituiert ist,
- A eine $C_1$-$C_{12}$-Alkoxygruppe ist,
- B ein Difluorbor-Beta-Diketon der Formel (III) ist

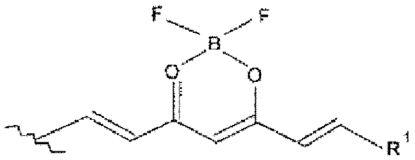

(III)

- n 0, 1, 2, 3 oder 4 ist,
- m 0 oder 1 ist oder

Q1 der Formel (IV) entspricht:

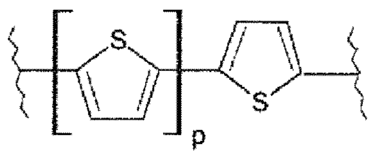

(IV)

wobei

- $Q^1$ mit $Q^2$ und $Q^3$ substituiert ist,
- p 0, 1 oder 2 ist,

▪ $Q^2$ ein Difluorbor-Beta-Diketon der Formel (V) ist

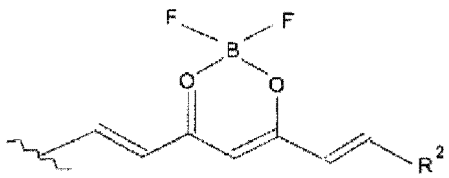

(V)

▪ $Q^3$ ein Difluorbor-Beta-Diketon der Formel (VI) ist

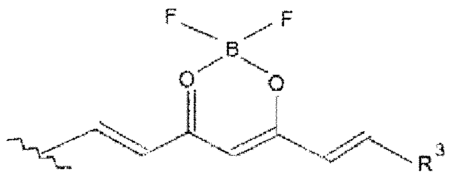

(VI)

- $R^1$, $R^2$ und $R^3$ jeweils unabhängig aus der nachfolgenden Gruppe gewählt sind:

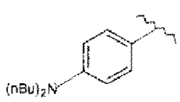, 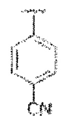, 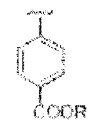, 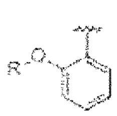, 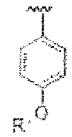, 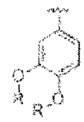, 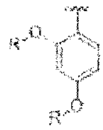, 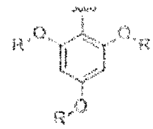,

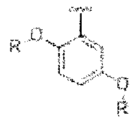, 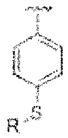, 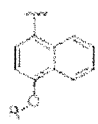, 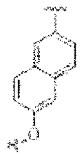, 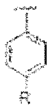, 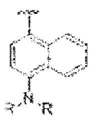, 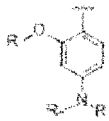, 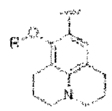, 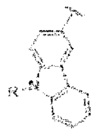,

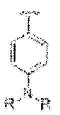, 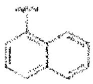, 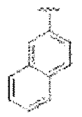, 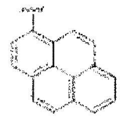, 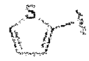, 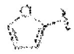, 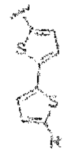, 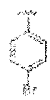, 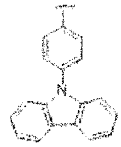,

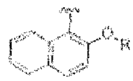, 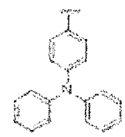

wobei R jeweils unabhängig ein Wasserstoffatom oder eine $C_1$-$C_{12}$-Alkylgruppe ist.

**2.** Verbindung der Formel (I) nach Anspruch 1, wobei:

- $Q^1$ der Formel (IIa) entspricht:

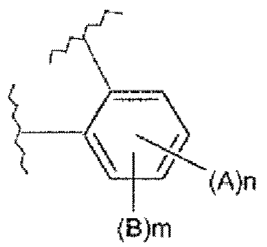

(IIa)

wobei

- $Q^1$ in der Ortho-Position mit $Q^2$ und $Q^3$ substituiert ist,
- A eine $C_1$-$C_{12}$-Alkoxygruppe ist,
- B ein Difluorbor-Beta-Diketon der Formel (III) ist

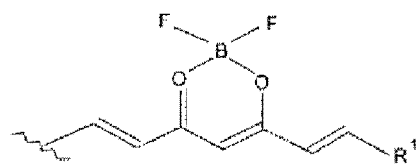

(III)

- n 0, 1, 2, 3 oder 4 ist,

- m 0 oder 1 ist; und

▪ $Q^2$, $Q^3$, R, $R^1$, $R^2$ und $R^3$ der Definition nach Anspruch 1 entsprechen.

**3.** Verbindung der Formel (I) nach Anspruch 1 oder 2, wobei:

▪ $Q^1$ der Formel (IIa) entspricht

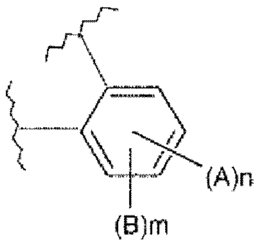

(IIa)

wobei

- $Q^1$ in der Ortho-Position mit $Q^2$ und $Q^3$ substituiert ist,
- n=m=0 und

$Q^2$ und $Q^3$ der Definition nach Anspruch 1 entsprechen, wobei $R^2$ und $R^3$ jeweils unabhängig aus der nachfolgenden Gruppe gewählt sind:

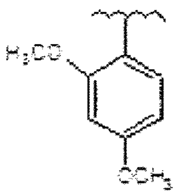
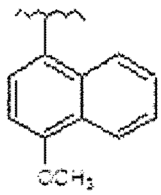
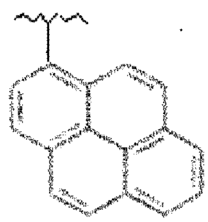
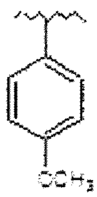

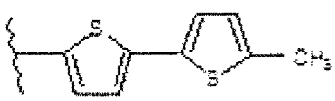
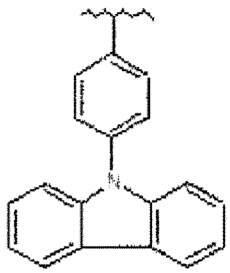
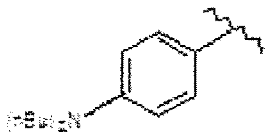

**4.** Verbindung der Formel (I) nach Anspruch 1, wobei:

▪ Q1 der Formel (IIb) entspricht:

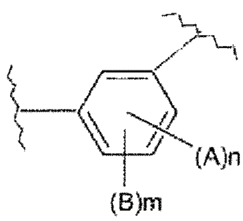

(IIb)

wobei

- $Q^1$ in der Meta-Position mit $Q^2$ und $Q^3$ substituiert ist,
- A eine $C_1$-$C_{12}$-Alkoxygruppe ist,
- B ein Difluorbor-Beta-Diketon der Formel (III) ist

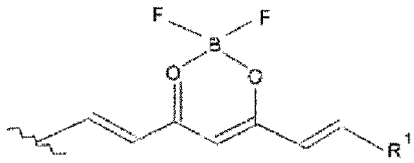
(III)

- n 0, 1, 2, 3 oder 4 ist,
- m 0 oder 1 ist; und

▪ $Q^2$, $Q^3$, R, $R^1$, $R^2$ und $R^3$ der Definition nach Anspruch 1 entsprechen.

**5.** Verbindung der Formel (I) nach Anspruch 1 oder 4, wobei:

▪ $Q^1$ der Formel (IIb) entspricht:

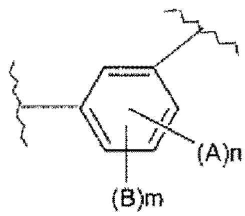
(IIb)

wobei

- $Q^1$ in der Meta-Position mit $Q^2$ und $Q^3$ substituiert ist,
- A eine $C_1$-$C_{12}$-Alkoxygruppe ist,
- B ein Difluorbor-Beta-Diketon der Formel (III) ist

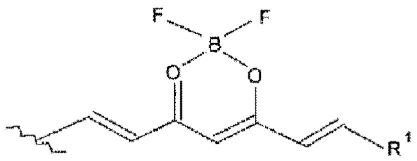
(III)

- n 0, 1, 2, 3 oder 4 ist,
- m 0 oder 1 ist; und

▪ $Q^2$ und $Q^3$ der Definition nach Anspruch 1 entsprechen, wobei $R^1$, $R^2$ und $R^3$ jeweils unabhängig aus der nachfolgenden Gruppe gewählt sind:

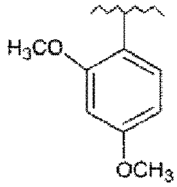 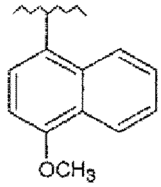 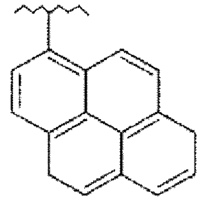 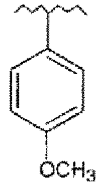

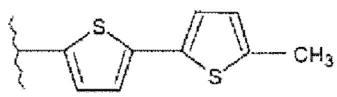
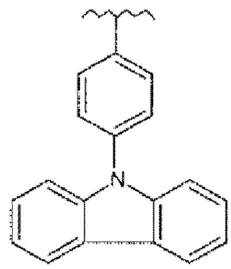
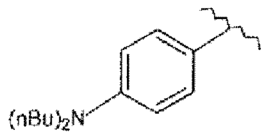

6. Verbindung der Formel (I) nach Anspruch 1, 4 oder 5, wobei:

- $Q^1$ der Formel (IIb) entspricht:

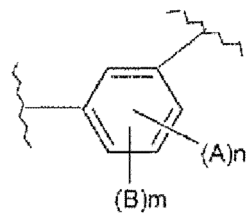

(IIb)

wobei

- $Q^1$ in der Meta-Position mit $Q^2$ und $Q^3$ substituiert ist,
- n= 1, 2, 3 oder 4,
- m=0,
- A eine $C_1$-$C_{12}$-Alkoxygruppe ist und

- $Q^2$ und $Q^3$ der Definition nach Anspruch 1 entsprechen, wobei $R^2$ und $R^3$ jeweils unabhängig aus der nachfolgenden Gruppe gewählt sind:

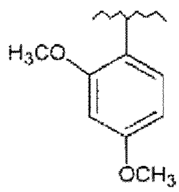
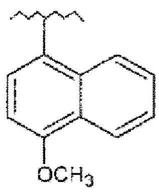
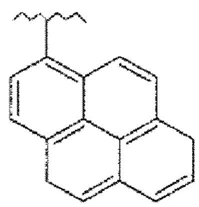
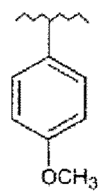

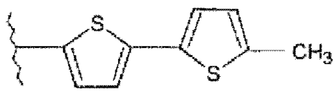
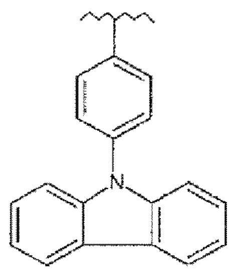
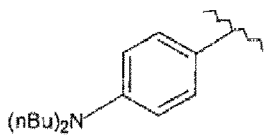

7. Verbindung der Formel (I) nach Anspruch 1, wobei:

- $Q^1$ der Formel (IIc) entspricht:

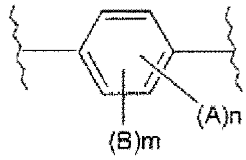

(IIc)

wobei

- $Q^1$ in der Para-Position mit $Q^2$ und $Q^3$ substituiert ist,
- A eine $C_1$-$C_{12}$-Alkoxygruppe ist,
- B ein Difluorbor-Beta-Diketon der Formel (III) ist

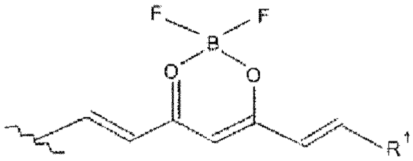

(III)

- n 0, 1, 2, 3 oder 4 ist,
- m 0 oder 1 ist; und

▪ $Q^2$, $Q^3$, R, $R^1$, $R^2$ und $R^3$ der Definition nach Anspruch 1 entsprechen.

**8.** Verbindung der Formel (I) nach Anspruch 1 oder 7, wobei:

▪ $Q^1$ der Formel (IIc) entspricht:

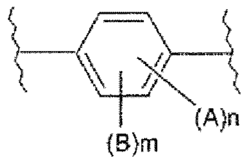

(IIc)

wobei

- $Q^1$ in der Para-Position mit $Q^2$ und $Q^3$ substituiert ist,
- A eine $C_1$-$C_{12}$-Alkoxygruppe ist,
- B ein Difluorbor-Beta-Diketon der Formel (III) ist

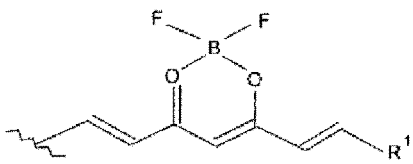

(III)

- n 0, 1, 2, 3 oder 4 ist,
- m gleich 0 oder 1 ist;

$Q^2$ und $Q^3$ der Definition nach Anspruch 1 entsprechen, wobei $R^1$, $R^2$ und $R^3$ jeweils unabhängig sind:

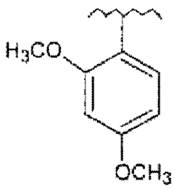 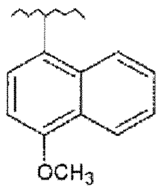 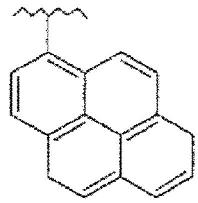 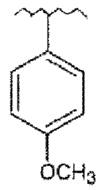

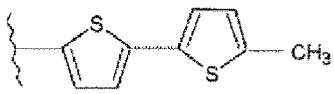 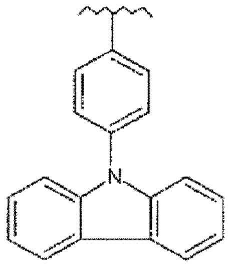 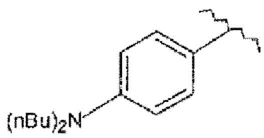

**9.** Verbindung der Formel (I) nach einem der Ansprüche 1, 7 und 8, wobei:

$Q^1$ der Formel (IIc) entspricht:

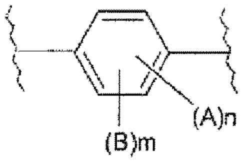
(IIc)

wobei

- $Q^1$ in der Para-Position mit $Q^2$ und $Q^3$ substituiert ist,
- A eine $C_1$-$C_{12}$-Alkoxygruppe ist,
- n 0, 1, 2, 3 oder 4 ist,
- m=0 und

$Q^2$ und $Q^3$ der Definition nach Anspruch 1 entsprechen, wobei $R^2$ und $R^3$ jeweils unabhängig aus der nachfolgenden Gruppe gewählt sind:

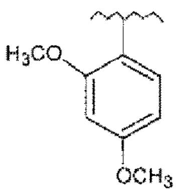 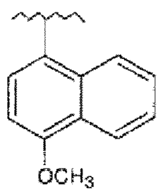 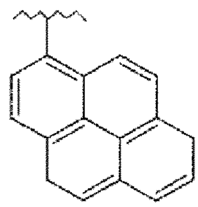 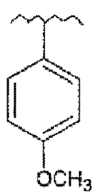

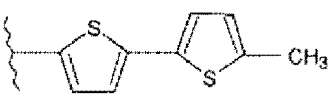 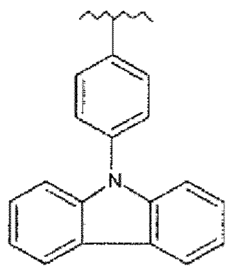 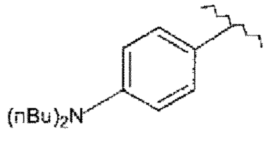

**10.** Verbindung der Formel (I) nach Anspruch 1, wobei:

$Q^1$ der Formel (IV) entspricht:

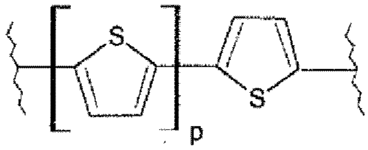

(IV)

wobei

- $Q^1$ mit $Q^2$ und $Q^3$ substituiert ist,
- p 0, 1 oder 2 ist und

  ▪ $Q^2$, $Q^3$, $R^2$ und $R^3$ der Definition nach Anspruch 1 entsprechen.

**11.** Verbindung der Formel (I) nach Anspruch 1 oder 10, wobei:

▪ $Q^1$ der Formel (IV) entspricht:

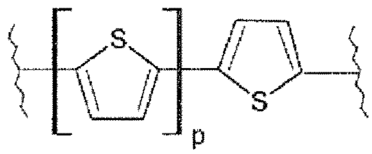

(IV)

wobei

- $Q^1$ mit $Q^2$ und $Q^3$ substituiert ist,
- p 0, 1 oder 2 ist und

▪ $Q^2$ und $Q^3$ der Definition nach Anspruch 1 entsprechen, wobei $R^2$ und $R^3$ jeweils unabhängig aus der nachfolgenden Gruppe gewählt sind:

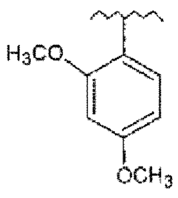
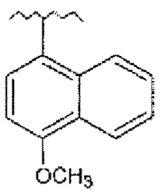
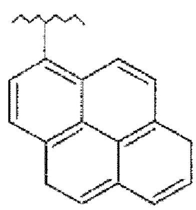
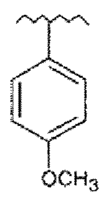
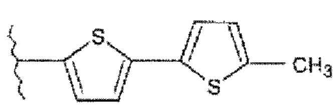
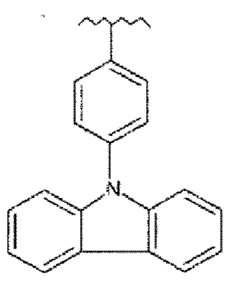
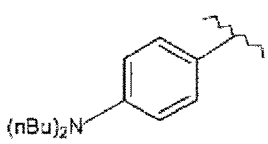

**12.** Verwendung einer Verbindung der Formel (I) nach einem der Ansprüche 1 - 11 als Fluorophor.

**13.** Verbindung der Formel (I) nach einem der Ansprüche 1 - 11 zur Verwendung bei Bioimaging, als Sensoren für

flüchtige Säuren/Basen, bei der photodynamischen Therapie, bei der Diagnose von M. Alzheimer, bei der Theranostik, als optische Sensoren für anaerobe Umgebungen, in der Anzeige- und Fernmeldetechnik, in der Photovoltaik.

**Revendications**

**1.** Composé de formule générale (I) :

$$Q^2\text{-}Q^1\text{-}Q^3 \qquad (I)$$

dans laquelle

• $Q^1$ est représenté par la formule (II)

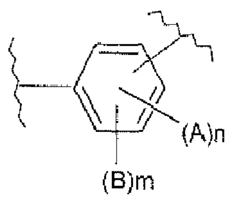

(II)

dans laquelle

- $Q^1$ est substitué par $Q^2$ et $Q^3$,
- A est un groupe alcoxy en $C_1$ à $C_{12}$,
- B est une difluorobore-bêta-dicétone de formule (III)

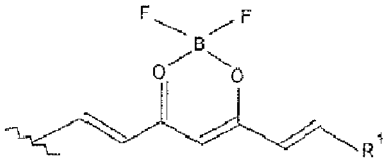

(III)

- n vaut 0, 1, 2, 3 ou 4,
- m vaut 0 ou 1 ; ou

• $Q^1$ est représenté par la formule (IV)

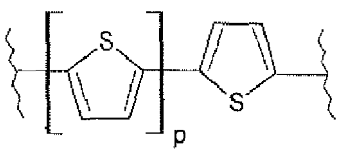

(IV)

dans laquelle

- $Q^1$ est substitué par $Q^2$ et $Q^3$,
- p vaut 0, 1 ou 2 ;

• $Q^2$ est une difluorobore-bêta-dicétone de formule (V)

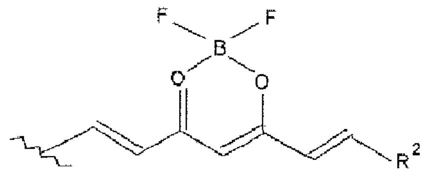

(V)

• $Q^3$ est une difluorobore-bêta-dicétone de formule (VI)

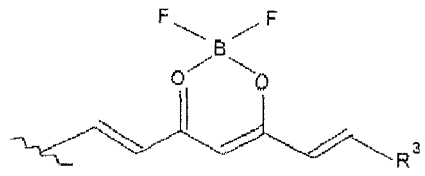

(VI)

• chacun de $R^1$, $R^2$ et $R^3$ est indépendamment choisi parmi :

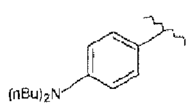 , 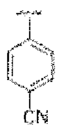 , 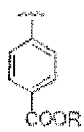 , 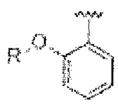 , 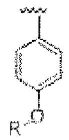 , 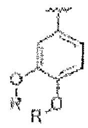 , 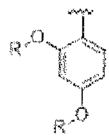 , 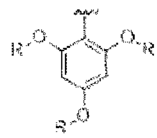 ,

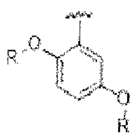 , 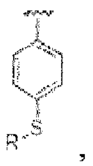 , 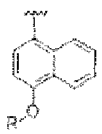 , 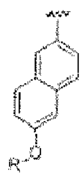 , 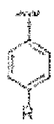 , 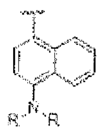 , 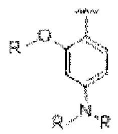 , 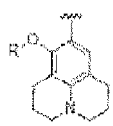 , 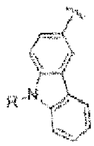 ,

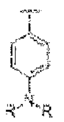 , 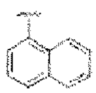 , 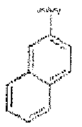 , 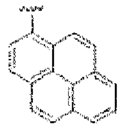 , 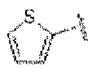 , 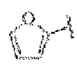 , 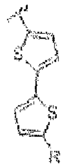 , 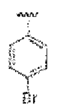 , 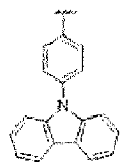 ,

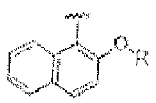 , 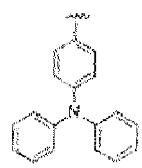

où chaque R est indépendamment un atome d'hydrogène, un groupe alkyle en $C_1$ à $C_{12}$.

**2.** Composé de formule (I) selon la revendication 1, dans lequel

• $Q^1$ est représenté par la formule (IIa)

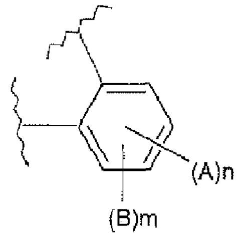

(IIa)

où

- Q1 est substitué par $Q^2$ et $Q^3$ en position ortho,
- A est un groupe alcoxy en $C_1$ à $C_{12}$,
- B est une difluorobore-bêta-dicétone de formule (III),

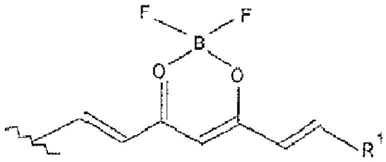

(III)

- n vaut 0, 1, 2, 3 ou 4,
- m vaut 0 ou 1 ; et

• $Q^2$, $Q^3$, R, $R^1$, $R^2$ et $R^3$ sont tels que définis dans la revendication 1.

**3.** Composé de formule (I) selon la revendication 1 ou 2, dans lequel

• $Q^1$ est représenté par la formule (IIa)

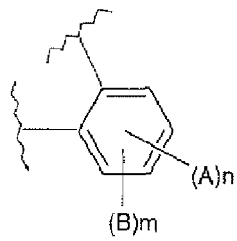

(IIa)

où

- Q1 est substitué par $Q^2$ et $Q^3$ en position ortho,
- n = m = 0 ; et

• $Q^2$ et $Q^3$ sont tels que définis dans la revendication 1, et chacun de $R^2$ et $R^3$ est indépendamment choisi parmi :

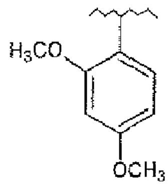
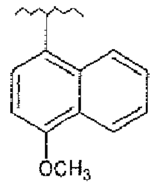
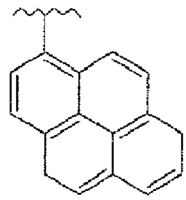
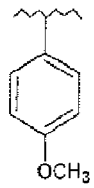

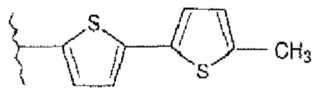
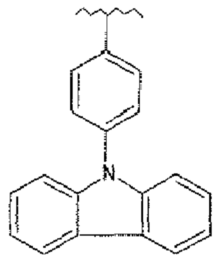
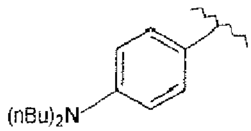

**4.** Composé de formule (I) selon la revendication 1, dans lequel

- $Q^1$ est représenté par la formule (IIb)

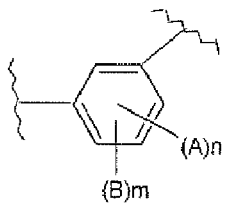

(IIb)

où

- Q1 est substitué par $Q^2$ et $Q^3$ en position méta,
- A est un groupe alcoxy en $C_1$ à $C_{12}$,
- B est une difluorobore-bêta-dicétone de formule (III),

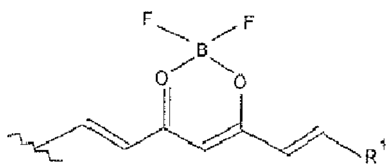

(III)

- n vaut 0, 1, 2, 3 ou 4,
- m vaut 0 ou 1 ; et

- $Q^2$, $Q^3$, R, $R^1$, $R^2$ et $R^3$ sont tels que définis dans la revendication 1.

**5.** Composé de formule (I) selon la revendication 1 ou 4, dans lequel

- $Q^1$ est représenté par la formule (IIb)

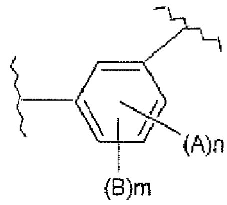

(IIb)

où

- Q1 est substitué par $Q^2$ et $Q^3$ en position méta,
- A est un groupe alcoxy en $C_1$ à $C_{12}$,
- B est une difluorobore-bêta-dicétone de formule (III),

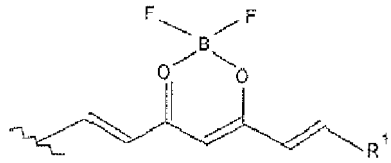

(III)

- n vaut 0, 1, 2, 3 ou 4,
- m vaut 0 ou 1 ; et

• $Q^2$ et $Q^3$ sont tels que définis dans la revendication 1, et chacun de $R^1$, $R^2$ et $R^3$ est indépendamment choisi parmi :

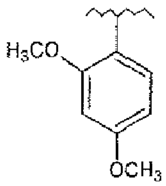 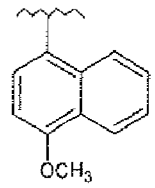 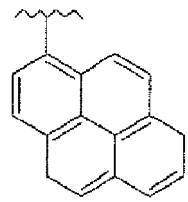 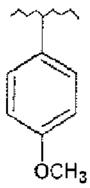

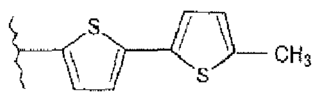 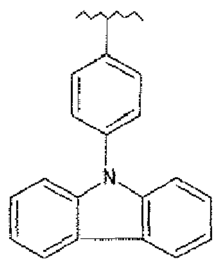 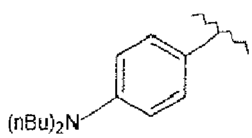

**6.** Composé de formule (I) selon l'une quelconque des revendications 1, 4 et 5, dans lequel

• $Q^1$ est représenté par la formule (IIb)

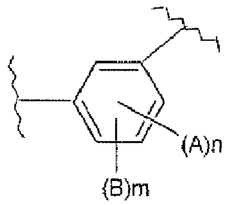

(IIb)

où

- $Q^1$ est substitué par $Q^2$ et $Q^3$ en position méta,
- n vaut 0, 1, 2, 3 ou 4,
- m = 0,
- A est un groupe alcoxy en $C_1$ à $C_{12}$ ; et

• $Q^2$ et $Q^3$ sont tels que définis dans la revendication 1, et chacun de $R^2$ et $R^3$ est indépendamment choisi parmi :

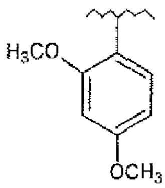
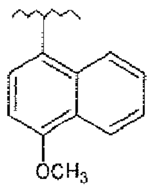
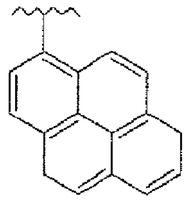
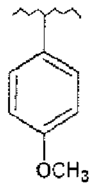

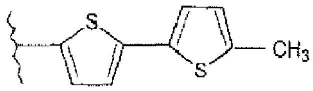
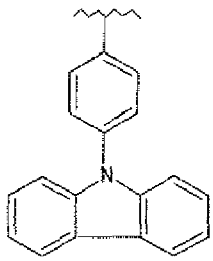
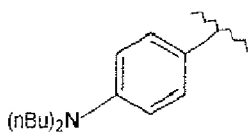

**7.** Composé de formule (I) selon la revendication 1, dans lequel

• $Q^1$ est représenté par la formule (IIc)

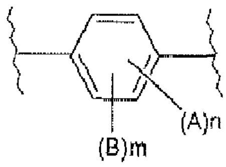

(IIc)

- $Q^1$ est substitué par $Q^2$ et $Q^3$ en position para,
- A est un groupe alcoxy en $C_1$ à $C_{12}$,
- B est une difluorobore-bêta-dicétone de formule (III),

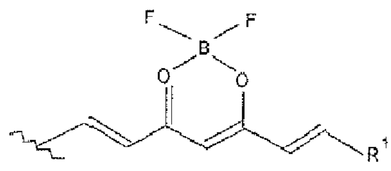

(III)

- n vaut 0, 1, 2, 3 ou 4,
- m vaut 0 ou 1 ; et

• $Q^2$, $Q^3$, R, $R^1$, $R^2$ et $R^3$ sont tels que définis dans la revendication 1.

**8.** Composé de formule (I) selon la revendication 1 ou 7, dans lequel

• $Q^1$ est représenté par la formule (IIc)

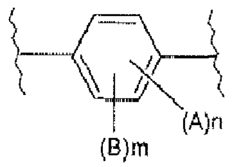

(IIc)

- $Q^1$ est substitué par $Q^2$ et $Q^3$ en position para,
- A est un groupe alcoxy en $C_1$ à $C_{12}$,
- B est une difluorobore-bêta-dicétone de formule (III),

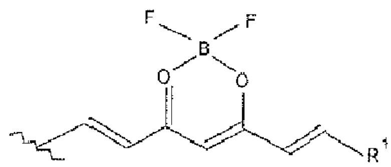

(III)

- n vaut 0, 1, 2, 3 ou 4,
- m vaut 0 ou 1 ;

• $Q^2$ et $Q^3$ sont tels que définis dans la revendication 1, et chacun de $R^1$, $R^2$ et $R^3$ est indépendamment choisi parmi :

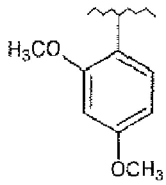
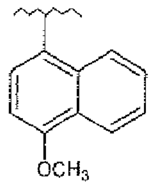
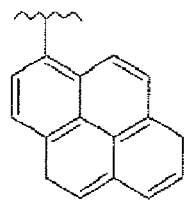
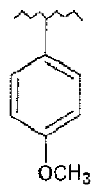

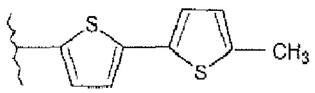
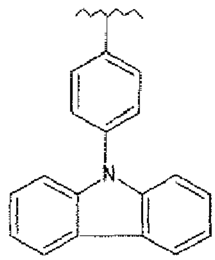
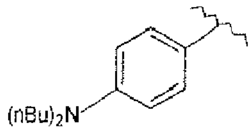

**9.** Composé de formule (I) selon l'une quelconque des revendications 1, 7 et 8, dans lequel

• $Q^1$ est représenté par la formule (IIc)

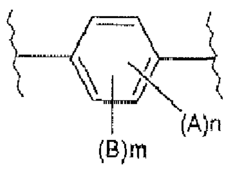

(IIc)

- Q1 est substitué par $Q^2$ et $Q^3$ en position para,
- A est un groupe alcoxy en $C_1$ à $C_{12}$,
- n vaut 0, 1, 2, 3 ou 4,
- m = 0 ; et

• $Q^2$ et $Q^3$ sont tels que définis dans la revendication 1, et chacun de $R^2$ et $R^3$ est indépendamment choisi parmi :

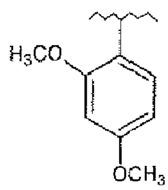
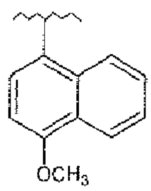
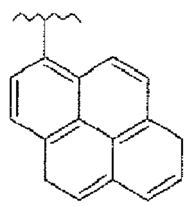
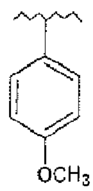

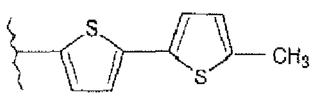
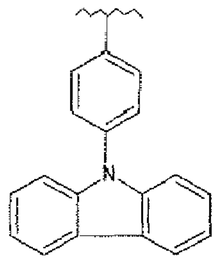
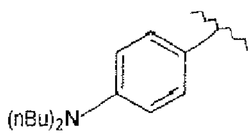

**10.** Composé de formule (I) selon la revendication 1, dans lequel

• $Q^1$ est représenté par la formule (IV)

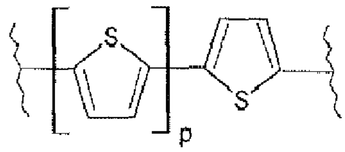

(IV)

où

- $Q^1$ est substitué par $Q^2$ et $Q^3$,
- p vaut 0, 1 ou 2 ; et

• $Q^2$, $Q^3$, $R^2$ et $R^3$ sont tels que définis dans la revendication 1.

**11.** Composé de formule (I) selon la revendication 1 ou 10, dans lequel

• $Q^1$ est représenté par la formule (IV)

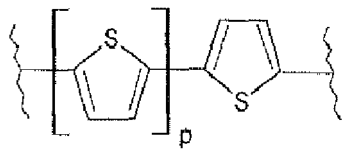

(IV)

où

- $Q^1$ est substitué par $Q^2$ et $Q^3$,
- p vaut 0, 1 ou 2 ; et

• $Q^2$ et $Q^3$ sont tels que définis dans la revendication 1, et chacun de $R^2$ et $R^3$ est indépendamment choisi parmi :

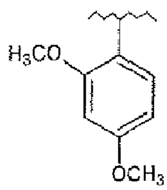
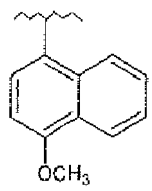
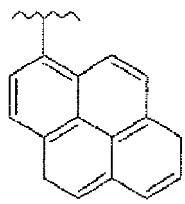
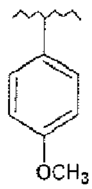

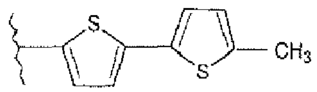
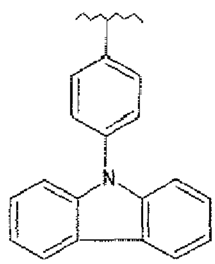
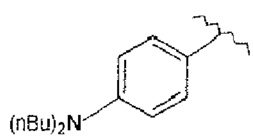

**12.** Utilisation d'un composé de formule (I) selon l'une quelconque des revendications 1 à 11 en tant que fluorophore.

**13.** Composé de formule (I) selon l'une quelconque des revendications 1 à 11 pour une utilisation en imagerie biologique, en tant que capteur d'acide/base volatil, en thérapie photodynamique, dans le diagnostic de la maladie d'Alzheimer,

en théranostique, en tant que capteur optique pour environnement anaérobie, dans les technologies de l'affichage et des télécommunications, dans les dispositifs photovoltaïques.

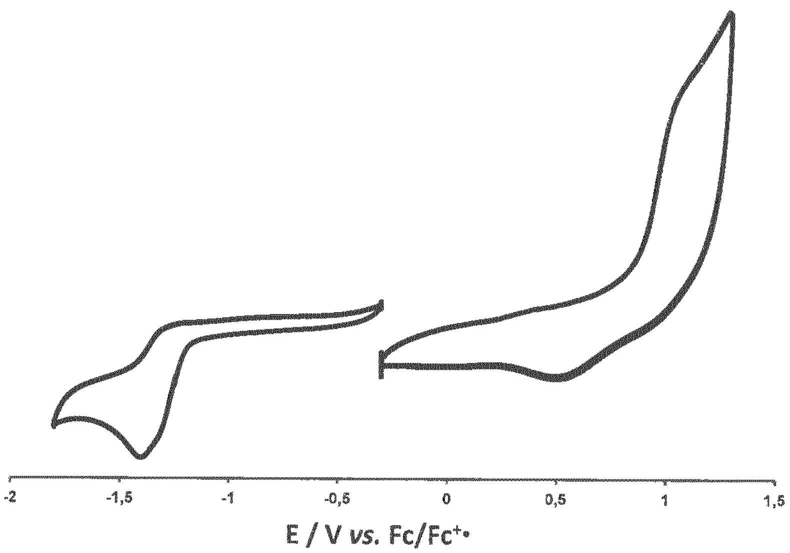


Figure 1

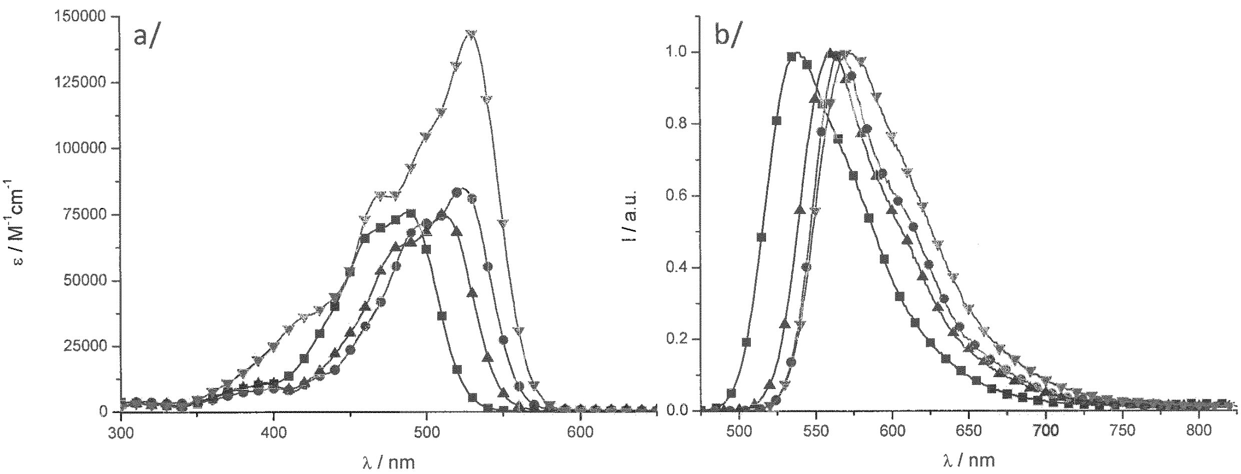
a/
b/
150000
125000
100000
75000
50000
25000
0
ε / M-1cm-1
300
400
500
600
λ / nm
1.0
0.8
0.6
0.4
0.2
0.0
I / a.u.
500
550
600
650
700
750
800
λ / nm

Figure 2

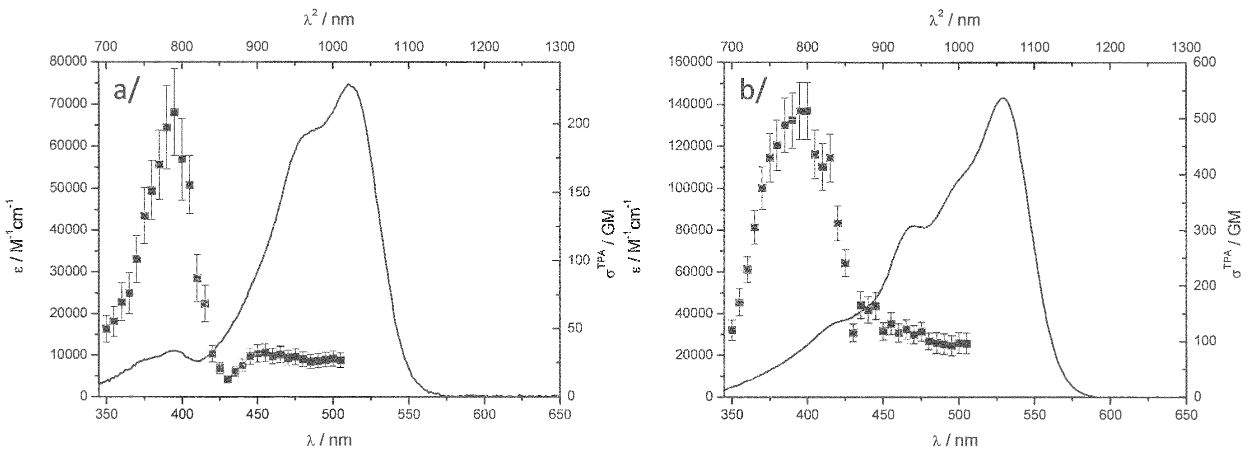
a/
b/
λ² / nm
λ / nm
ε / M⁻¹cm⁻¹
σᵀᴾᴬ / GM
700
800
900
1000
1100
1200
1300
350
400
450
500
550
600
650
80000
70000
60000
50000
40000
30000
20000
10000
0
200
150
100
50
160000
140000
120000
100000
80000
60000
40000
20000
600
500
400
300
200
100

Figure 3

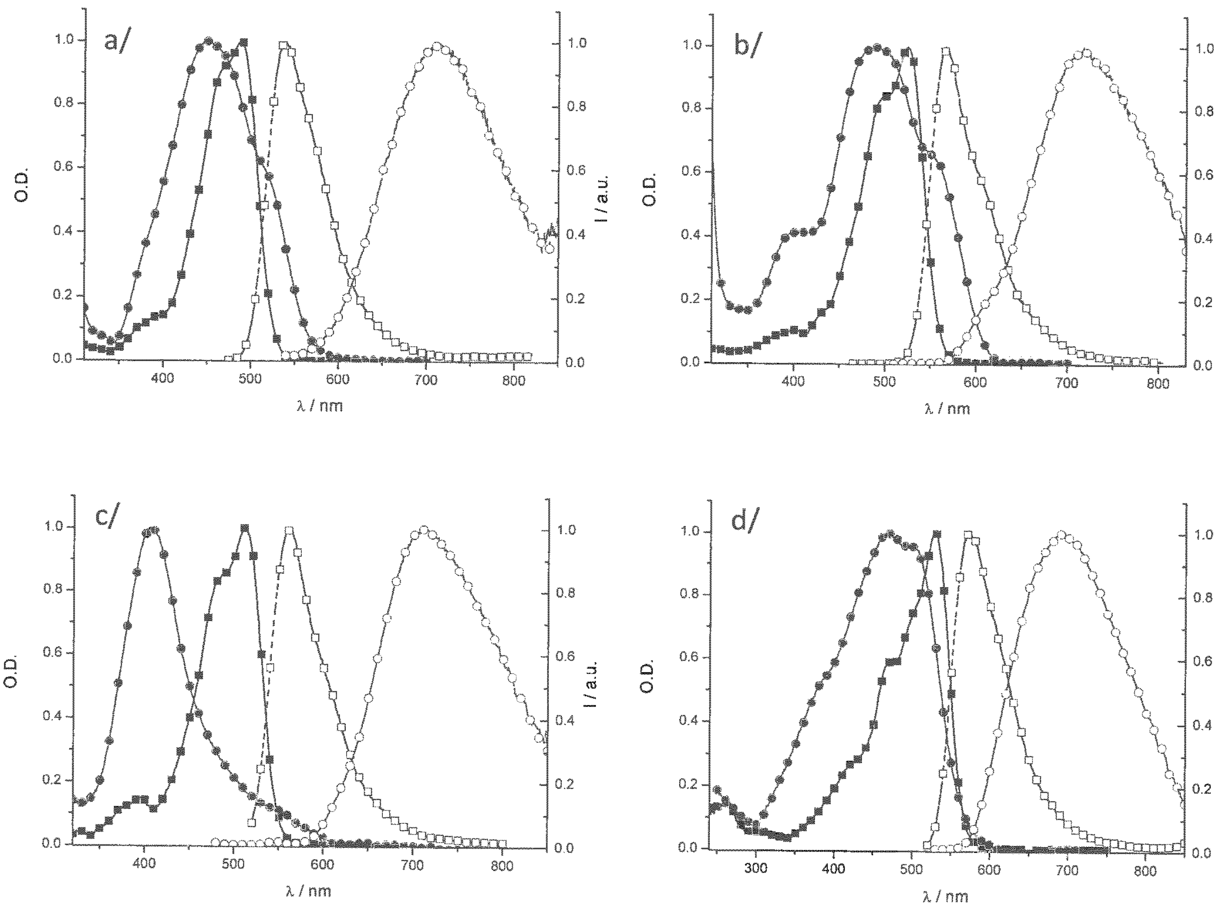


Figure 4

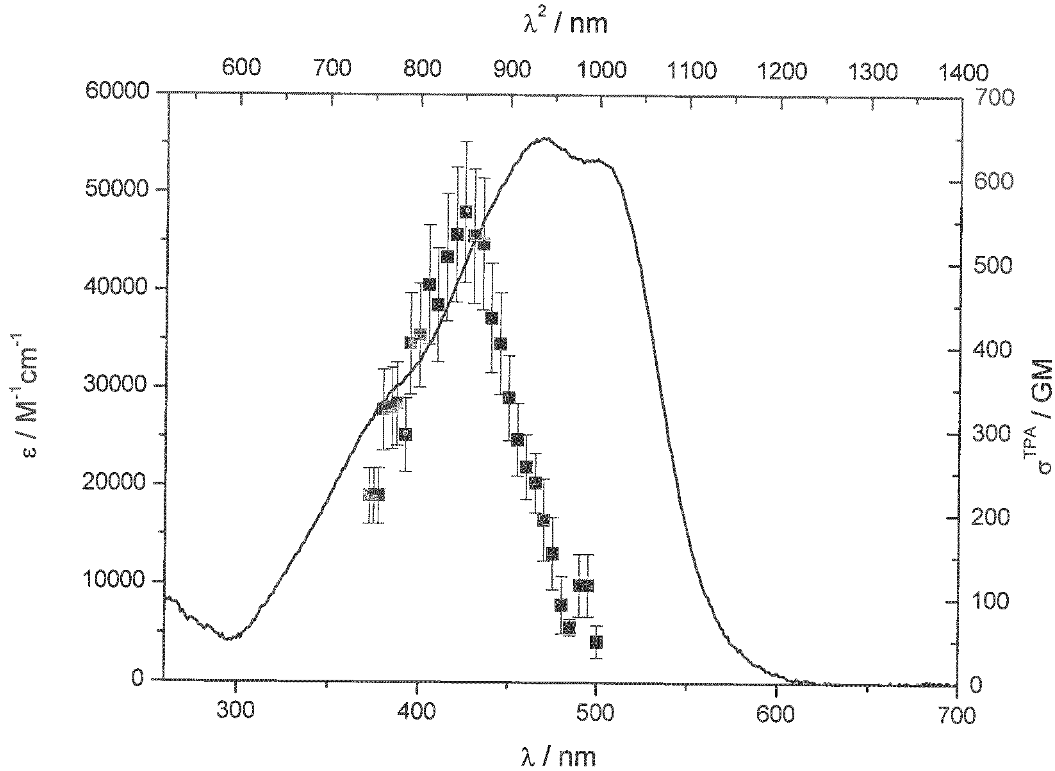
λ² / nm
600
700
800
900
1000
1100
1200
1300
1400
60000
50000
40000
30000
20000
10000
0
ε / M⁻¹cm⁻¹
700
600
500
400
300
200
100
0
σTPA / GM
300
400
500
600
700
λ / nm

**Figure 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **A. D'ALÉO ; D. GACHET ; V. HERESANU ; M. GIORGI ; F. FAGES.** *Chem. Eur. J.,* 2012, vol. 18, 12764-12772
- **G. BAI ; C. YU ; C. CHENG ; E. HAO ; Y. WEI ; X. MU ; L. JIAO.** *Org. Biomol. Chem.,* 2014, vol. 12, 1618-1626
- **HUALONG FU et al.** *J. Med.Chem.,* 2015, vol. 58, 6972-6983
- **G. MANN ; L. BEYER ; A. ARRIETA.** *Z. Chem.,* 1987, vol. 27, 172-173
- *J. Med. Chem.,* 2006, vol. 49, 6111-6119
- **C. A. PARKER.** Photoluminescence of Solutions. Elsevier Publishing, 1969
- **J. R. LAKOWICZ.** Principles of Fluorescence Spectroscopy. Kluwer, 2006
- **N. G. CONNELLY ; W. E. GEIGER.** *Chem. Rev.,* 1996, vol. 96, 877-910
- **C. XU ; W. W. WEBB.** *J. Opt. Soc. Am. B,* 1996, vol. 13, 481-491
- **H. KAWAI ; H. S. NALWA ; H. OIKAWA ; S. OKADA ; H. MATSUDA ; N. MINAMI ; A. KAKUDA ; K. ONO ; A. MUKOH ; H. NAKANISHI.** *Jpn. J. Appl. Phys.,* 1992, vol. 31, L1132-L1134
- **A. FELOUAT ; A. D'ALÉO ; F. FAGES.** *J. Org. Chem.,* 2013, vol. 78, 4446-4455